(19) Europäisches Patentamt
European Patent Office
Office européen des brevets

(11) Veröffentlichungsnummer **0 055 216**
**B1**

(12) # EUROPÄISCHE PATENTSCHRIFT

(45) Veröffentlichungstag der Patentschrift: **19.09.84**

(51) Int. Cl.³: **C 09 D 7/12**, C 08 K 5/34, C 07 D 211/14, C 07 D 211/46, C 07 D 211/58

(21) Anmeldenummer: **81810497.8**

(22) Anmeldetag: **14.12.81**

(54) **Verfahren zur Härtung von Einbrennlacken.**

(30) Priorität: **19.12.80 CH 9408/80**

(43) Veröffentlichungstag der Anmeldung: **30.06.82 Patentblatt 82/26**

(45) Bekanntmachung des Hinweises auf die Patenterteilung: **19.09.84 Patentblatt 84/38**

(84) Benannte Vertragsstaaten: **CH DE FR GB IT LI**

(56) Entgegenhaltungen:
**EP - A - 0 006 213**
**US - A - 3 474 054**

(73) Patentinhaber: **CIBA-GEIGY AG, Postfach, CH-4002 Basel (CH)**

(72) Erfinder: **Karrer, Friedrich, Dr., Rebbergstrasse 5, CH-4800 Zofingen (CH)**
Erfinder: **Berner, Godwin, Dr., Waldhofstrasse 70, CH-4310 Rheinfelden (CH)**
Erfinder: **Rody, Jean, Dr., Rütiring 82, CH-4125 Riehen (CH)**

**EP 0 055 216 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die Erfindung betrifft ein Verfahren zur Härtung von sauer härtbaren Einbrennlacken unter Verwendung von Säureadditions-Salzen basischer Polyalkylpiperidinderivate oder innerer Salze von Polyalkylpiperidinsulfonsäuren.

Einbrennlacke sind Lacke, die durch Temperaturbehandlung gehärtet werden können. Sie basieren auf Bindemitteln die in der Lage sind, beim Einbrennen Vernetzungsreaktionen einzugehen. Solche Bindemittel können beispielsweise Acryl-, Polyester-, Alkyd-, Phenol-, Melamin-, Harnstoff-, Epoxid- oder Polyurethanharze sein. Es kann sich dabei auch um Gemische zweier verschiedener Harze, z. B. eines Polyester- und eines Melaminharzes handeln oder um Gemische eines reaktiven Harzes und einem monomeren Vernetzer, z. B. ein Acrylharz mit einem Glykol-diacrylat, oder um Harze, die verschiedene reaktive Gruppen in sich enthalten wie z. B. Acrylat-modifizierte Polyester. Die zur Vernetzung befähigten reaktiven Gruppen können olefinische Doppelbindungen sein, die unter Copolymerisation vernetzen, oder es handelt sich um Gruppen, die zur Polykondensation befähigt sind. Letztere Gruppen dominieren bei den heute gebräuchlichen Einbrennlacken und trotz verschiedenem chemischem Härtungsmechanismus haben diese Polykondensationsharze gemeinsam, daß ihre Härtung durch saure Katalysatoren beschleunigt werden kann. Der Vorteil des Zusatzes von Härtungskatalysatoren ist die Verkürzung der notwendigen Härtungszeiten oder die Herabsetzung der notwendigen Härtungstemperaturen. Der Nachteil des Katalysatorzusatzes ist die Herabsetzung der Lagerbeständigkeit, da in Gegenwart solcher Katalysatoren bereits bei Raumtemperatur eine langsame Polykondensation einsetzt. Der saure Katalysator kann also erst kurz vor der Applikation dem Lack zugesetzt werden und der so katalysierte Lack muß innerhalb bestimmter Topfzeiten appliziert werden. Aus diesem Grunde werden Härtungskatalysatoren nur in speziellen Fällen verwendet, beispielsweise bei Automobil-Reparaturlacken, wo ein Einbrennen bei hohen Temperaturen nicht möglich ist.

Als saure Katalysatoren verwendet man in diesen Fällen schwer-flüchtige Säuren wie z. B. Phosphorsäure, aromatische Sulfonsäuren oder Maleinsäurehalbester. Es wurde auch bereits vorgeschlagen, statt der freien Säuren deren Salze mit organischen Aminen zu verwenden. Beispielsweise schlägt das US-Patent 34 74 054 Pyridinsalze von p-Toluolsulfonsäure und die DE-OS 29 20 306 Oxazolidinsalze von aromatischen Sulfonsäuren als Härtungskatalysatoren vor. Solche Salze sind bei Raumtemperatur annähernd neutral, beim Erhitzen spalten sie das flüchtige Amin ab, die nicht-flüchtige Säure verbleibt im Lack und bewirkt dort eine rasche Härtung. Man kann solche Salze daher als latente saure Härtungskatalysatoren bezeichnen. Ihr Vorteil ist eine beträchtliche Verlängerung der Topfzeit, ihr Nachteil ist jedoch eine Geruchsbelästigung während des Härtungsprozesses durch das sich verflüchtigende Amin. Da Amine physiologisch nicht unbedenklich sind, muß man die Einbrennvorrichtungen mit wirkungsvollen Exhaustoren ausstatten und das abgesaugte Luftgemisch muß gereinigt werden. Es besteht daher ein Bedarf nach latenten Härtungskatalysatoren, die beim Einbrennen keine Amindämpfe abgeben und trotzdem lange Topfzeiten gewährleisten.

Es wurde gefunden, daß man als Härtungskatalysatoren auch Säureadditionssalze von wenig- oder nicht-flüchtigen Aminen verwenden kann. Dadurch ist es möglich, Salze von solchen Aminen zu verwenden, die als Lichtschutzmittel bekannt sind wie z. B. Salze von Derivaten von Polyalkylpiperidinen. Da diese Amine beim Einbrennprozeß sich nicht verflüchtigen, verbleiben sie als Lichtschutzmittel im Lack.

Solche Salze wirken also aufgrund ihres Säure-Anions als Härtungskatalysatoren für sauer härtbare Lackharze und aufgrund ihres Kations als Lichtschutzmittel für die gehärteten Lacke.

Gegenstand der Erfindung ist daher ein Verfahren zur Härtung von Einbrennlacken durch Erwärmen in Gegenwart eines Härtungskatalysators, das dadurch gekennzeichnet ist, daß man als Härtungskatalysator entweder a) ein Säureadditionssalz eines basischen Polyalkylpiperidinderivates, das eine Gruppe der Formel I enthält,

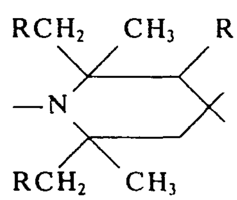

(I)

worin R Wasserstoff oder Methyl ist, und einer anorganischen oder organischen Säure, oder b) das innere Salz einer Sulfonsäure, die eine Gruppe der Formel I enthält, verwendet. In Formel I ist R bevorzugt Wasserstoff.

Als Einbrennlacke kommen hierfür solche Einbrennlacke in Frage, deren Härtung durch saure Katalysatoren beschleunigt werden kann. Das sind vor allem Lacke auf Basis von Acryl-, Polyester-, Alkyd-, Melamin- und Phenolharzen, insbesondere aber die Mischungen von Acryl-, Polyester- oder Alkydharzen untereinander oder mit einem Melaminharz. Darunter fallen auch modifizierte Lackharze wie z. B.

acrylmodifizierte Polyester- oder Alkydharze. Beispiele für einzelne Typen von Harzen, die unter den Begriff Acryl-, Polyester- und Alkydharze fallen, sind z. B. in Wagner, Sarx/Lackkunstharze (München, 1971) Seiten 86–123 und 229–238, oder in Ullmann/Encyclopädie der techn. Chemie, 4. Auflage, Band 15 (1978), Seiten 613–628, beschrieben. Von besonderer Bedeutung ist die saure Katalyse für die Härtung von Lacken, die veretherte Melaminharze enthalten, wie z. B. methylierte oder butylierte Melaminharze (N-Methoxymethyl- bzw. N-Butoxymethylmelamine).

Polyalkylpiperidinderivate, die die Gruppe der Formel I enthalten, sind als Lichtschutzmittel bekannt. Es kann sich dabei um Mono-, Oligo- oder Polypiperidine handeln, je nachdem, ob die Verbindungen der Gruppe der Formel I in ihrem Molekül einmal oder mehrmals enthalten sind oder ob es sich um Polymere mit wiederkehrenden Einheiten der Formel I handelt. Die meisten dieser Piperidinderivate enthalten an der 4-Stellung des Piperidinderivates einen polaren Substituenten, beispielsweise eine Ether-, Ester-, Amid-, Carbamat-, Ketal-, Amino- oder Triazinylfunktion. Die wichtigsten und erfindungsgemäß bevorzugten Klassen von Polyalkylpiperidinen sind die folgenden Verbindungsklassen:

a) 4-Hydroxypiperidine und deren Ether, Ester und Carbamate der Formel

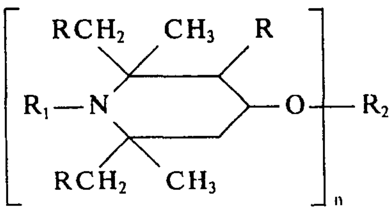

worin R Wasserstoff oder Methyl ist, $R_1$ Alkyl, Hydroxyalkyl, Cyanoalkyl, Alkenyl, Alkinyl oder Aralkyl, vorzugsweise $C_1$–$C_4$ Alkyl, Allyl oder Benzyl bedeutet, n 1 bis 4, vorzugsweise 1 oder 2 ist, und $R_2$, wenn n = 1 ist, Wasserstoff, Alkyl, Cyanethyl, Benzyl, Glycidyl, einen Monoacylrest einer gesättigten oder ungesättigten aliphatischen, cycloaliphatischen, araliphatischen oder aromatischen Mono- oder Dicarbonsäure, Carbaminsäure oder Phosphor enthaltenden Säure oder einen einwertigen Silylrest, vorzugsweise einen Rest einer aliphatischen Monocarbonsäure mit 2 bis 18 C-Atomen oder einer aromatischen Monocarbonsäure mit 7 bis 15 C-Atomen, wenn n = 2 ist, Alkylen, Alkenylen, Xylylen, einen Diacylrest einer gesättigten oder ungesättigten aliphatischen, cycloaliphatischen, araliphatischen oder aromatischen Dicarbonsäure, Dicarbaminsäure oder Phosphor enthaltenden Säure oder einen zweiwertigen Silylrest, vorzugsweise einen Rest einer aliphatischen Dicarbonsäure mit 4–14 C-Atomen, einer aromatischen Dicarbonsäure mit 8–14 C-Atomen, einer aliphatischen, cycloaliphatischen oder aromatischen Dicarbaminsäure mit 8–14 C-Atomen, wenn n = 3 ist, einen Triacylrest einer aliphatischen, cycloaliphatischen oder aromatischen Tricarbonsäure, einer aromatischen Tricarbaminsäure oder einer Phosphor enthaltenden Säure oder einen dreiwertigen Silylrest und, wenn n = 4 ist, einen Tetraacylrest einer aliphatischen, cycloaliphatischen oder aromatischen Tetracarbonsäure bedeutet.

b) 4-Aminopiperidine und ihre N-Acylderivate der Formel

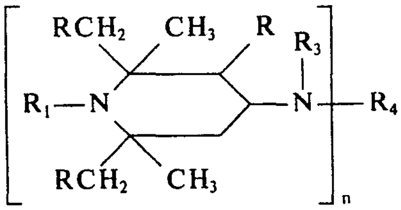

worin n die Zahlen 1 oder 2 bedeutet, R und $R_1$ die unter a) angegebene Bedeutung haben, $R_3$ Alkyl, Cycloalkyl, Aralkyl, Alkanoyl, Alkenoyl oder Benzoyl ist und $R_4$, wenn n = 1 ist, Alkyl, Cycloalkyl, gegebenenfalls mit einer Cyano-, Carbonyl- oder Carbamidgruppe substituiertes Alkenyl, Glycidyl, eine Gruppe der Formel

$$-CH_2-CH(OH)-Z$$

ist, worin Z Wasserstoff, Methyl oder Phenyl bedeutet, oder $R_3$ und $R_4$ zusammen den cyclischen Rest einer aliphatischen oder aromatischen 1,2- oder 1,3-Dicarbonsäure bedeuten und, wenn n = 2 ist, $R_4$ Alkylen, Xylylen, eine $-CH_2-CH(OH)-CH_2$-Gruppe oder eine Gruppe

$$-CH_2CH(OH)-CH_2-O-X-O-CH_2-CH(OH)-$$

bedeutet, worin X Alkylen, Arylen, Cycloalkylen ist, oder, vorausgesetzt, daß $R_3$ nicht Alkanoyl, Alkenoyl oder Benzoyl bedeutet, $R_4$ auch einen zweiwertigen Rest einer aliphatischen, cycloaliphatischen oder aromatischen Dicarbonsäure oder Dicarbaminsäure bedeuten kann.

c) Cyclische Ketale von 4-Oxopiperidinen der Formel

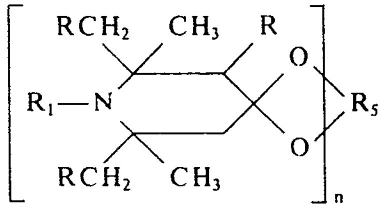

worin n die Zahlen 1 oder 2 bedeutet, R und $R_1$ die unter a) angegebene Bedeutung haben und $R_5$, wenn n = 1 ist, Alkylen oder Hydroxyalkylen, Acyloxyalkylen und, wenn n = 2 ist, die Gruppe $(-CH_2)_2C(CH_2-)_2$ bedeutet.

d) Spirohydantoine der Formel

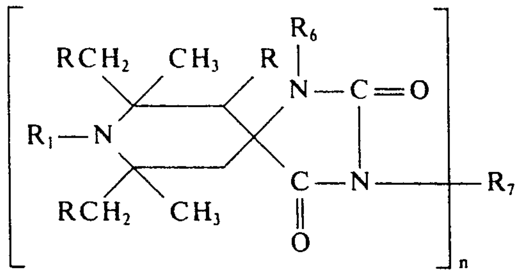

worin n 1 oder 2 ist, R und $R_1$ die unter a) angegebene Bedeutung haben, $R_6$ Wasserstoff, Alkyl, Allyl, Benzyl, Glycidyl oder Alkoxyalkyl ist, und $R_7$, wenn n = 1 ist, Wasserstoff, Alkyl, Glycidyl, Alkenyl, Aralkyl, Cycloalkyl, Hydroxyalkyl, Alkoxyalkyl, Aryl und, wenn n = 2 ist, Alkylen, Arylen oder eine Gruppe

$$-CH_2-CH(OH)-CH_2-O-X-O-CH_2-CH(OH)-CH_2-$$

bedeutet, worin X die unter b) angegebene Bedeutung hat.

e) Triazinderivate der Formel

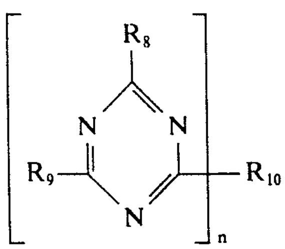

worin n 1, 2 oder 3 ist und $R_8$ eine Gruppe der Formel

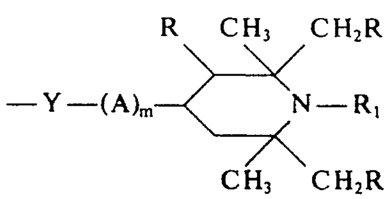

bedeutet, worin R und $R_1$ die unter a) angegebene Bedeutung haben, Y –O– oder $-NR_{11}-$ ist, A Alkylen und m 0 oder 1 bedeuten, $R_9$ dieselbe Bedeutung wie $R_8$ hat oder eine der Gruppen $-NR_{11}R_{12}$ oder $-OR_{13}$ ist, $R_{10}$, wenn n = 1 ist, dieselbe Bedeutung wie $R_8$ oder $R_9$ hat, wenn n = 2 ist, eine Gruppe –Y–Q–Y– ist, worin Q gegebenenfalls durch –O– oder $-N(R_{14})-$ unterbrochenes Alkylen bedeutet, und wenn n = 3 ist, eine Gruppe

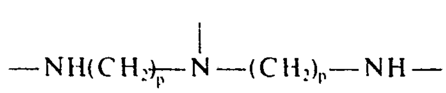

bedeutet, worin p 2 oder 3 ist, $R_{11}$ Alkyl, Cyclohexyl, Benzyl oder Hydroxyalkyl oder eine Gruppe der Formel

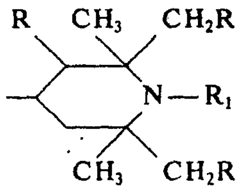

ist, $R_{12}$ Alkyl, Cyclohexyl, Benzyl oder Hydroxyalkyl, oder $R_{11}$ und $R_{12}$ zusammen $C_4$–$C_5$-Alkylen oder Oxaalkylen sind oder auch $R_{11}$ und $R_{12}$ jeweils eine Gruppe der Formel

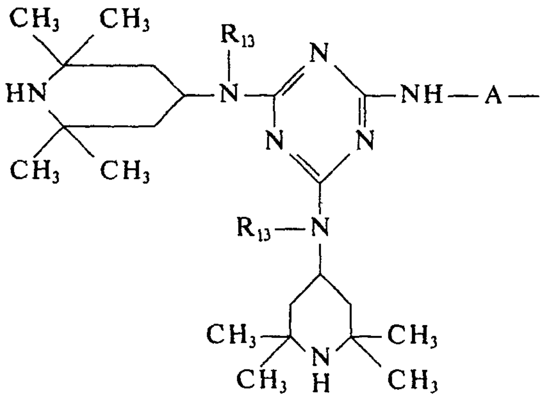

bedeuten, $R_{13}$ Wasserstoff, Alkyl oder Phenyl und $R_{14}$ Wasserstoff oder $C_1$–$C_4$ Alkyl ist.

f) Spirooxazolidone der Formel

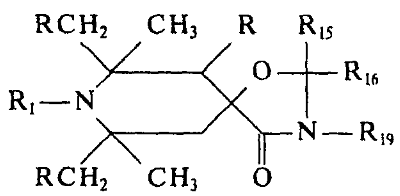

oder

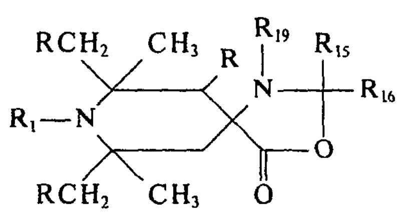

oder

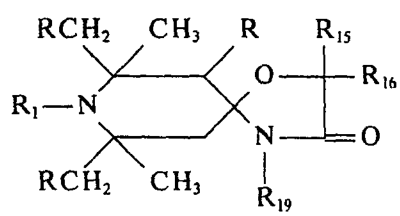

worin R und $R_1$ die unter a) gegebene Bedeutung haben, $R_{15}$ und $R_{16}$ unabhängig voneinander Wasserstoff, Alkyl, Aryl, Aralkyl oder Cycloalkyl oder $R_{15}$ und $R_{16}$ zusammen $C_4$–$C_{11}$ Alkylen bedeuten und $R_{19}$ Wasserstoff, Alkyl, Allyl oder Benzyl ist.

g) Bispiperidine der Formel

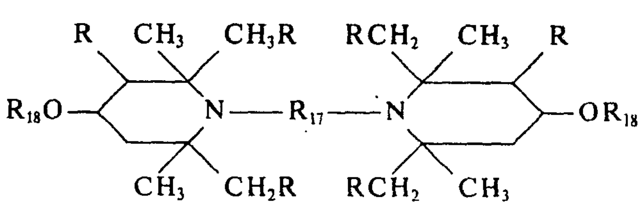

worin $R_{17}$ Alkylen, Alkenylen oder Aralkylen, vorzugsweise 2-Butenylen-1,4 oder p-Xylylen ist und $R_{18}$ Wasserstoff, Allyl, Benzyl oder eine Gruppe $-CO-R_{19}$ oder $-CO-R_{20}-COOH$ ist, worin $R_{19}$ Alkyl, Aryl oder Cyclohexyl und $R_{20}$ Alkylen oder Alkenylen bedeutet.

h) Polymere Verbindungen, deren wiederkehrende Struktureinheiten eine Gruppe der Formel I enthalten, wobei diese ein wiederkehrender Bestandteil der Polymer-Hauptkette oder Bestandteil einer wiederkehrenden Seitengruppe sein kann, insbesondere Polyester, Polyether, Polyamide, Polyamine, Polyurethane, Polyharnstoffe, Polyaminotriazine, Poly(meth)acrylate, Poly(meth)acrylamide und deren Copolymeren, die solche Reste enthalten.

Bevorzugt sind Säureadditionssalze von Polyalkylpiperidinderivaten, welche eine Gruppe der Formel Ia enthalten,

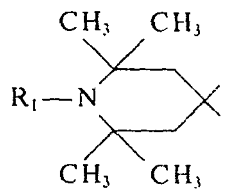

(Ia)

worin $R_1$ $C_1$–$C_4$ Alkyl, Cyanomethyl, $C_3$–$C_5$ Alkenyl oder $C_7$–$C_9$ Aralkyl, insbesondere Allyl oder Benzyl bedeutet.

Beispiele für Säuren, die mit den Piperidinderivaten Salze im erfindungsgemäßen Sinn bilden, sind anorganische Säuren wie z. B. Phosphorsäure oder Amidosulfonsäure, vorzugsweise jedoch organische Säuren, wie Mono- oder Polysulfonsäuren, beispielsweise Methansulfonsäure, Methylamidosulfonsäure, Ethansulfonsäure, 2-Ethylhexansulfonsäure, Vinylsulfonsäure, Benzolsulfonsäure, Toluol- oder Naphthalinsulfonsäuren, Benzol- oder Naphthalin-disulfonsäuren, Styrolsulfonsäure, alkylierte Benzol- oder Naphthalinsulfonsäuren, technische Gemische von Paraffinsulfonsäuren, Acrylamido-N-alkansulfonsäuren, Polystyrolpolysulfonsäuren oder Polyvinylsulfonsäure; saure Phosphorsäureester wie z. B. Phenylphosphorsäure oder Diethylphosphorsäure; Phosphon- und Phosphinsäuren wie z. B. Methylphosphonsäure, Phenylphosphinsäure, Dodecylphenylphosphonsäure, Phenyl-butylphosphinsäure, Diphenylphosphinsäure oder Methyl-benzyl-phosphinsäure; Phosphonsäuremonoester wie z. B. Methyl-methylphosphonat oder Ethylphenylphosphonat; Maleinsäure und deren Monoester wie z. B. Monobutylmaleat oder Monohexylmaleat; und Phthalsäure und deren Monoester wie z. B. Monohexylphthalat oder Monooctylphthalat.

Bevorzugt sind die Salze von Mono- und Polysulfonsäuren, von sauren Phosphorsäureestern, von Phosphonsäuren und deren Monoestern, von Maleinsäure und deren Monoestern und von Phthalsäure und deren Monoestern.

Besonders bevorzugt sind die Säureadditionssalze von Polyalkylpiperidinderivaten, die eine Gruppe der Formel I enthalten, worin R Wasserstoff ist, mit einer Mono- oder Disulfonsäure.

Die Salze können neutrale Salze sein, d. h. sie enthalten gleich viel Basenäquivalente wie Säureäquivalente. Die Salze können aber auch saure oder basische Salze sein, wobei also entweder die Säureäquivalente oder die basischen Äquivalente im Überschuß sind. Bevorzugt sind neutrale Salze oder Salze mit einem geringen Überschuß (1–20%) an Basenäquivalenten.

Für eine hohe Lagerbeständigkeit der mit diesen Salzen katalysierten Lacke ist eine gewisse Basizität der Salze, wie sie bei Überschuß an Basenäquivalenten resultiert, von Vorteil. Für eine rasche Härtung in der Hitze ist ein Überschuß an Base jedoch von Nachteil. Je nach dem Verwendungszweck der Lacke kann man daher die Basizität entsprechend variieren. Zur Erhöhung der Basizität kann nicht nur die dem Salz zugrundeliegende Piperidinbase im Überschuß verwendet werden, man kann auch durch Zugabe geringer Mengen anderer Aminbasen die Basizität des Härtungskatalysators erhöhen und dadurch die Lagerstabilität des Lackes erhöhen.

Die Salze können auf bekannte Weise durch Neutralisation der Piperidinbase mit einer Säure hergestellt werden. Dies geschieht vorzugsweise durch Mischen von Lösungen der beiden Komponenten. Ist das Salz in dem verwendeten Lösungsmittel unlöslich oder schwerlöslich, so fällt es oder kristallisiert es aus der Lösung aus. Andernfalls isoliert man das Salz durch Verdampfen des Lösungsmittels. Für die erfindungsgemäße Verwendung zur Härtung von Lacken können aber auch die resultierenden Lösungen verwendet werden oder man konzentriert die Lösung durch teilweises Abdestillieren des Lösungsmittels auf eine bestimmte Konzentration.

Als organische Salze sind diese Piperidinsalze nicht nur in Wasser sondern auch in organischen Lösungsmitteln löslich. Dies ist wichtig für ihre Anwendung in Lacken, da diese meist organische Lösungsmittel enthalten. Die Löslichkeit der Salze hängt von ihrer Konstitution ab, insbesondere von der Art des Säurerestes (Anions). Dasselbe gilt für die Konsistenz der Salze. Diese können ölige Sirupe oder niedrigschmelzende amorphe Festkörper oder höher-schmelzende kristalline Festkörper sein. Soweit sie Festkörper sind, schmelzen sie unscharf, da sie beim Erwärmen teilweise in Base und Säure dissoziieren, bei deren Schmelzpunkt ein teilweises Erweichen des Salzes zu beobachten ist.

Bei Raumtemperatur liegen die Salze größtenteils in protonierter Form vor. Anhand einer Einzelverbindung kann man dies beispielhaft durch folgende zwei Formeln ausdrücken:

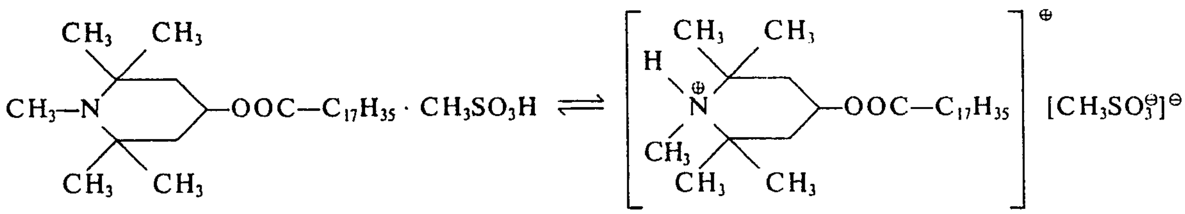

Wenn im folgenden die einfachere Schreibweise gemäß der linken Formel verwendet wird, so bedeutet dies trotzdem, daß der Großteil des Salzes in der Form der rechten Formel vorliegen kann.

Beispiele für Säureadditionssalze von Polyalkylpiperidinen, wie sie erfindungsgemäß verwendet werden können, sind die Verbindungen der folgenden Formeln, wobei

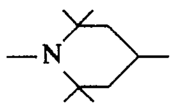

einen Rest der Formel

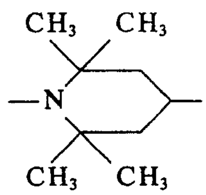

bedeuten soll.

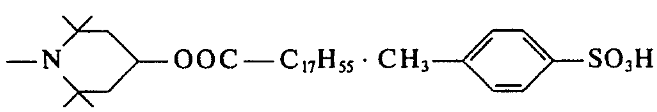

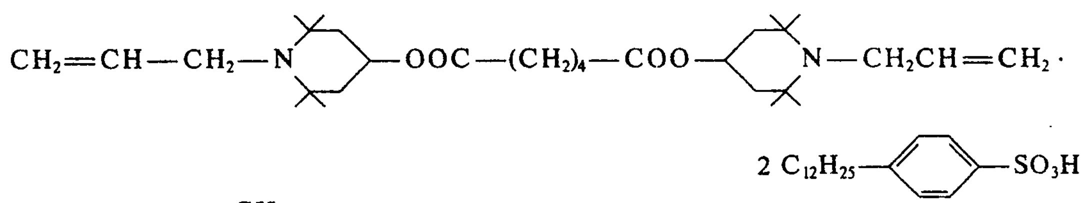

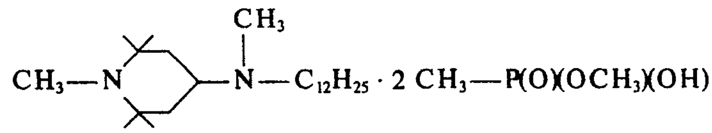

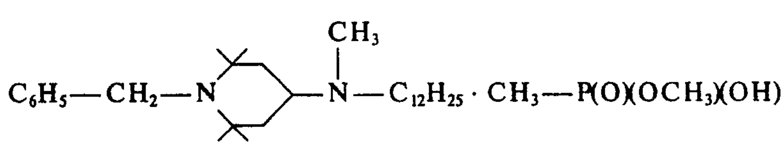

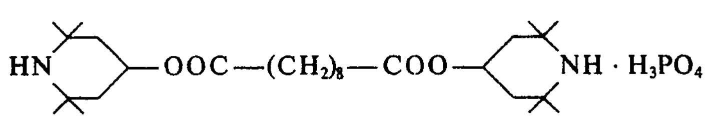

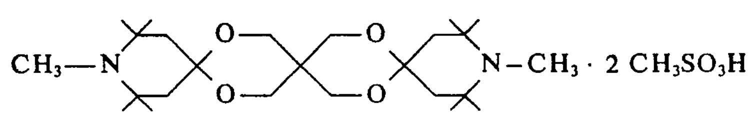

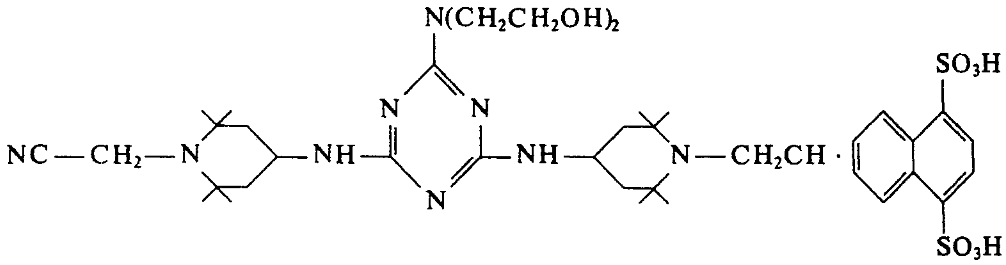

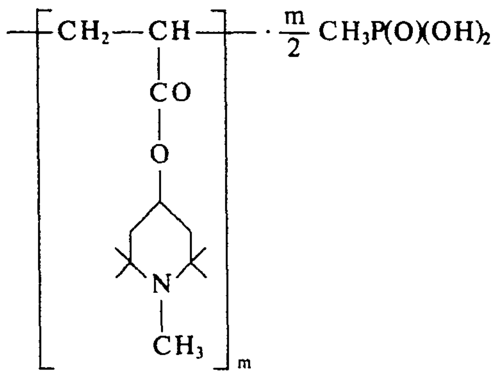

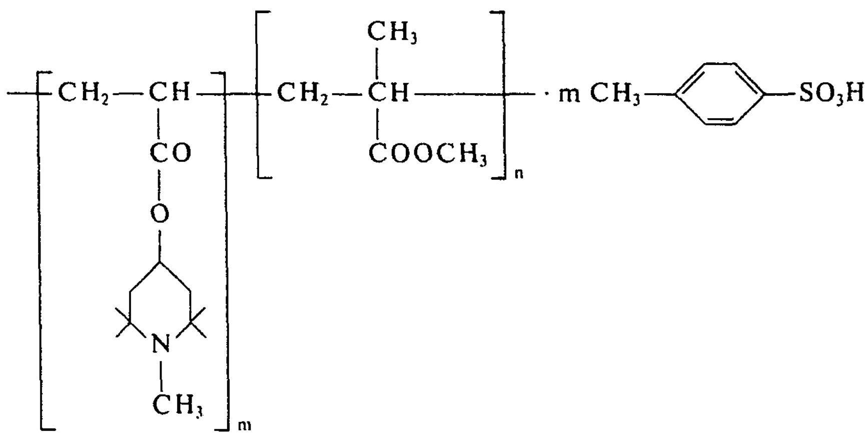

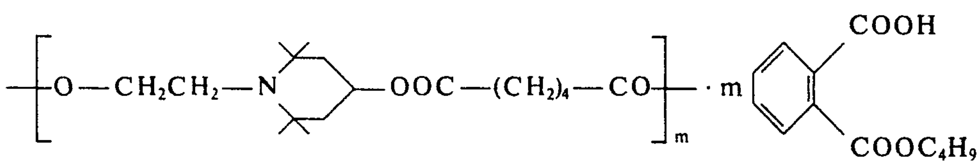

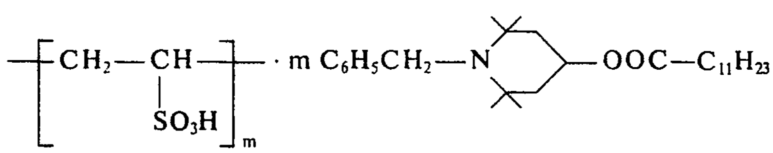

Anstelle dieser Säureadditionssalze können auch innere Salze von Sulfonsäuren, die eine Gruppe der Formel I enthalten, verwendet werden, insbesondere von Sulfonsäuren der folgenden allgemeinen Formeln II bis VII,

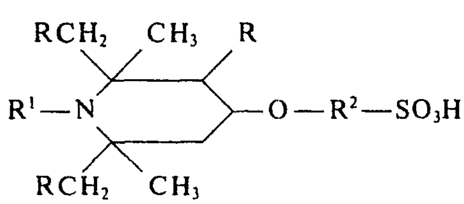

(II)

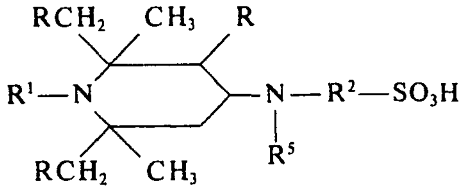

(III)

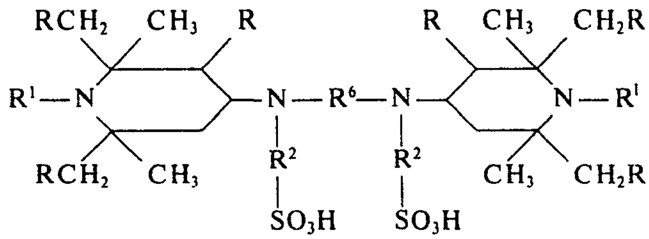

(IV)

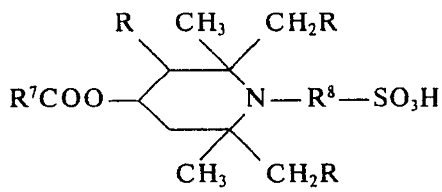

(V)

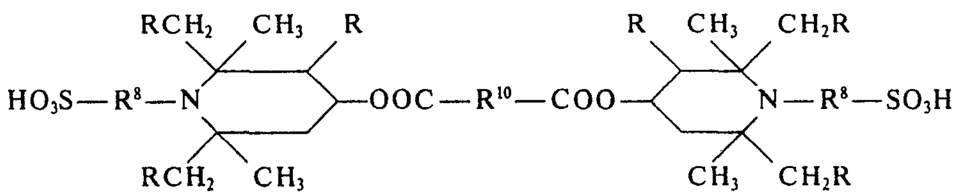

(VI)

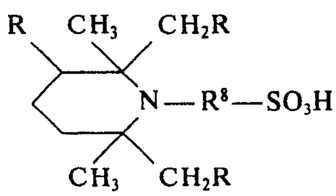

(VII)

worin R Wasserstoff oder Methyl ist,

$R^1$ Wasserstoff, $C_1$—$C_{12}$ Alkyl, $C_3$—$C_8$ Alkenyl, $C_3$—$C_8$ Alkinyl, $C_7$—$C_{12}$ Aralkyl oder $-CH_2CN$ ist,

$R^2$ eine Gruppe $-(CH_2)_3-$ oder $-CH_2-CH(R^3)-CONH-R^4$ darstellt, worin

$R^3$ Wasserstoff oder Methyl und $R^4$ $C_2$—$C_8$ Alkylen bedeutet,

$R^5$ $C_1$—$C_{12}$ Alkyl, $C_5$—$C_7$ Cycloalkyl oder $C_7$—$C_9$ Aralkyl,

$R^6$ $C_2$—$C_{12}$ Alkylen, durch —O— unterbrochenes $C_4$—$C_{10}$ Alkylen, $C_6$—$C_{15}$ Cycloalkylen oder Xylylen,

$R^7$ $C_1$—$C_{18}$ Alkyl, $C_6$—$C_{12}$ Aryl oder Alkaryl, $C_7$—$C_{12}$ Aralkyl oder eine Gruppe $R^{11}-NH-$,

$R^8$ $-(CH_2)_3-$ oder $-CH_2-CH(R^9)-O-(CH_2)_3-$,

$R^9$ Wasserstoff, Methyl, Phenyl, Phenoxymethyl oder Tolyloxymethyl,

$R^{10}$ $C_2$—$C_{12}$ Alkylen, durch —O— unterbrochenes $C_2$—$C_{12}$ Alkylen, $C_6$—$C_{12}$ Arylen, $C_6$—$C_{12}$ Cycloalkylen oder eine Gruppe $-NH-R^{12}-NH-$,

$R^{11}$ $C_1$—$C_{18}$ Alkyl, Cyclohexyl, $C_6$—$C_{12}$ Aryl oder $C_7$—$C_9$ Aralkyl und

$R^{12}$ $C_2$—$C_{12}$ Alkylen, $C_6$—$C_{14}$ Arylen oder $C_6$—$C_{12}$ Cycloalkylen

bedeuten.

Bevorzugt sind darunter die inneren Salze von Sulfonsäuren der Formel II, worin $R^1$ Methyl, Allyl oder Benzyl ist und $R^2$ eine Gruppe $-(CH_2)_3-$ darstellt.

Diese inneren Salze von Polyalkylpiperidinsulfonsäuren liegen teilweise in einer sogenannten Betainform vor, bei welcher sich das Proton der Säuregruppe am basischen Stickstoffatom befindet. Es herrscht ein Gleichgewicht zwischen beiden Grenzformen, die am Beispiel einer einzelnen Verbindung folgendermaßen dargestellt werden kann:

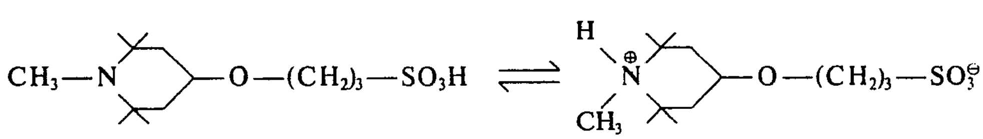

Die Lage des Gleichgewichtes hängt von der Art des Lösungsmittels ab und von der Temperatur.

Die Polyalkylpiperidinsulfonsäuren der Formeln II bis VII sind neue Verbindungen, die außer ihrer Verwendung als Härtungskatalysatoren für Lacke auch als Lichtschutzmittel für verschiedene organische Materialien, beispielsweise für Kunststoffe, Kosmetika oder photographische Schichten, verwendet werden können.

Die Herstellung der Verbindungen der Formeln II bis IV, worin $R^2$ eine Gruppe $-(CH_2)_3-$ ist, kann durch Reaktion der entsprechenden Piperidine, die in 4-Stellung eine OH- oder NH-Gruppe besitzen, mit Propansulton geschehen, beispielsweise für Verbindungen der Formel II nach folgendem Schema:

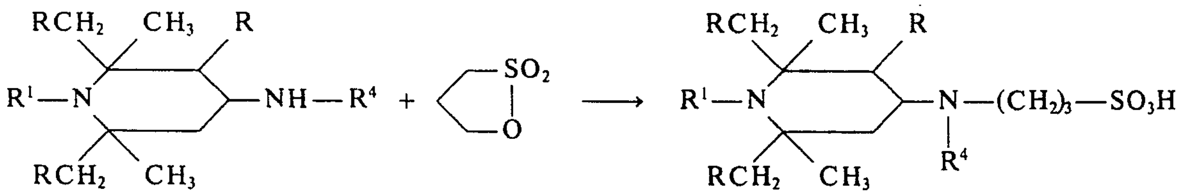

Ist in den Verbindungen der Formeln II–IV $R^2$ eine Gruppe

$$-CH_2-CH(R^3)-CONH-R^4$$

so können diese aus den entsprechenden OH- oder NH-Verbindungen durch Reaktion mit einer (Meth)acrylamidoalkylsulfonsäure der Formel VIII

$$CH_2{=}C(R^3)-CONH-R^4-SO_3H \qquad \text{(VIII)}$$

hergestellt werden.

Verbindungen der Formel V bis VII, worin $R^8$ eine Gruppe $-(CH_2)_3-$ ist, können aus den entsprechenden in 1-Stellung unsubstituierten Piperidinen durch Reaktion mit Propansulton hergestellt werden, beispielsweise für Verbindungen der Formel V nach folgendem Schema:

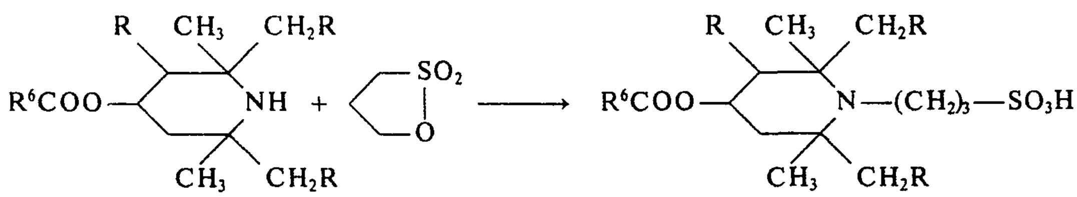

In analoger Weise können die Verbindungen der Formel V bis VII, worin $R^8$ eine Gruppe

$$-CH_2-CH(R^9)-O-(CH_2)_3-$$

ist, durch Umsetzung der entsprechenden 1-Hydroxyalkylpiperidine mit Propansulton hergestellt werden.

Beispiele für erfindungsgemäß verwendbare Polyalkylpiperidinsulfonsäuren sind die Verbindungen der folgenden Formeln, wobei

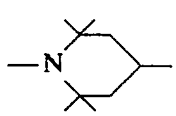

einen Rest der Formel

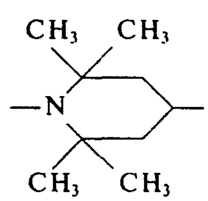

bedeutet.

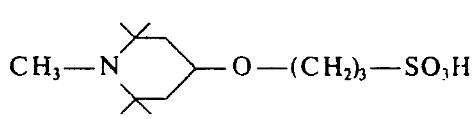

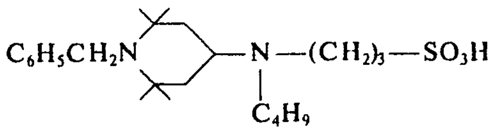

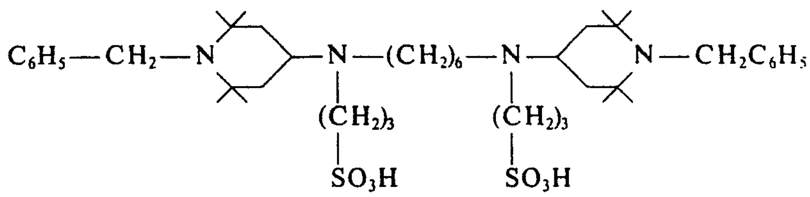

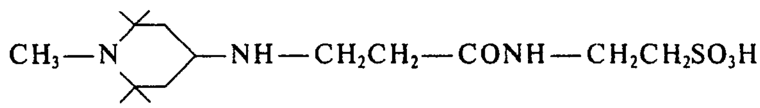

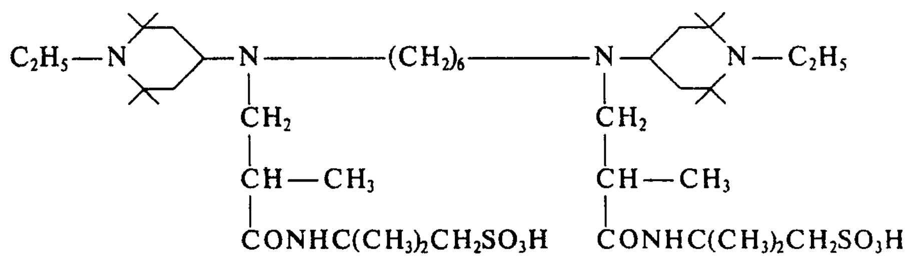

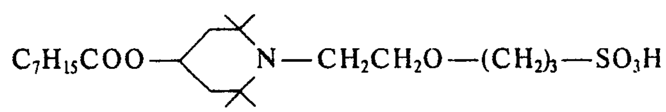

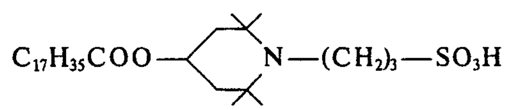

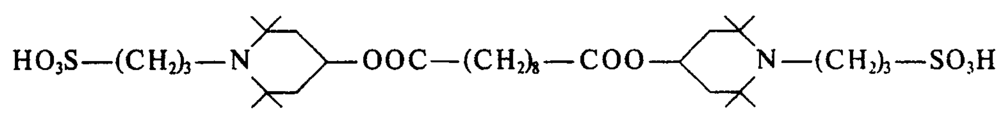

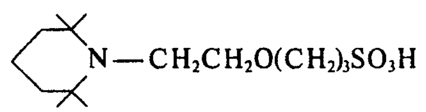

Im folgenden wird die Herstellung von Säureadditionssalzen von Polyalkylpiperidinderivaten sowie von inneren Salzen von Polyalkylpiperidinsulfonsäuren nach verschiedenen Methoden beispielhaft beschrieben und werden einzelne solcherart hergestellte Salze beschrieben. Die Temperaturen sind hierin in °C angegeben.

Herstellung von Säureadditionssalzen-Methode A

12,6 g Di(1-allyl-2,2,6,6-tetramethyl-4-piperidyl)-adipat (0,025 Mol), gelöst in 125 ml Acetonitril, werden mit einer Lösung von 9,5 g p-Toluolsulfonsäure-monohydrat (0,05 Mol) in 70 ml Acetonitril vermischt, das Lösungsmittel im Vakuum abdestilliert und der Rückstand aus Ethylenglykoldimethylether kristallisiert. Die Kristalle werden abfiltriert, mit wenig Hexan gewaschen und im Vakuum getrocknet. Das erhaltene Salz der Formel

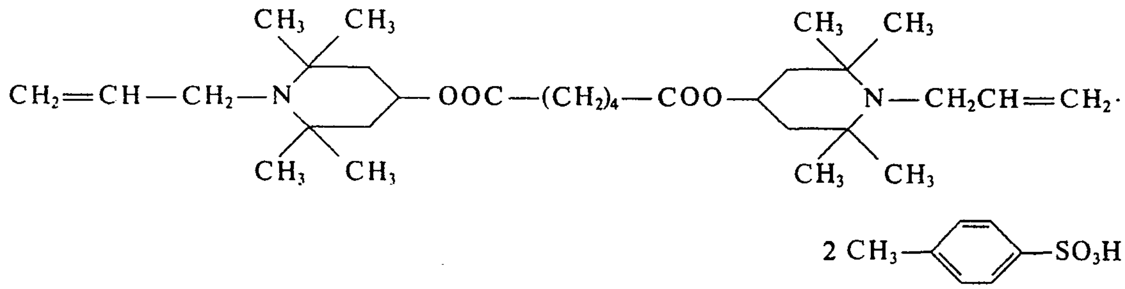

schmilzt bei 196–197 °C.

### Methode B

Eine Lösung von 9,63 g Di(1,2,2,6,6-pentamethyl-4-piperidyl)-sebacat in 50 ml Methylenchlorid wird mit einer Lösung von 11,84 g technischer Dodecylbenzolsulfonsäure (Äquivalenz-Gewicht 329) in 100 ml Hexan vermischt. Die resultierende Lösung wird im Vakuum eingedampft und der sirupöse Rückstand in 20,6 g Xylol gelöst, wodurch eine 50%ige Lösung des Salzes der Formel

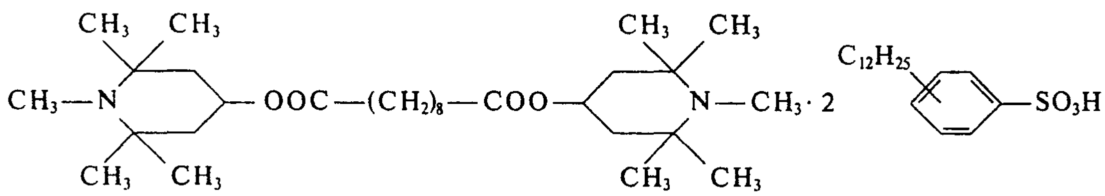

erhalten wird.

### Methode C

Eine Lösung von 11,5 g (0,02 Mol) Di(1-allyl-2,2,6,6-tetramethyl-4-piperidyl)-sebacat in 100 ml Aceton wird unter Rühren mit 19,1 g (0,02 Mol) einer 50%igen Lösung von technischer Dinonylnaphthalindisulfonsäure in Isobutanol (titriertes Äquivalenzgewicht der Lösung 478) vermischt. Die resultierende trübe Lösung wird filtriert und im Vakuum zur Trockne eingedampft. Das so erhaltene Salz der Formel

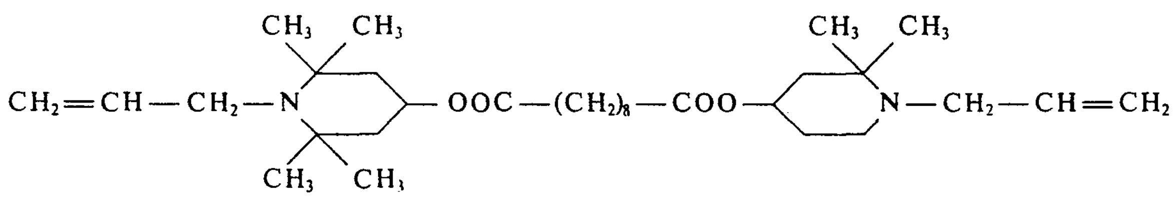

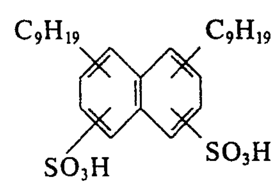

stellt ein gelbliches Pulver dar, das bei 104 °C erweicht.

### Analog hergestellte Salze

In der folgenden Aufstellung werden Salze beschrieben, die nach den oben beschriebenen Methoden hergestellt wurden. Hierbei werden die folgenden Abkürzungen verwendet:

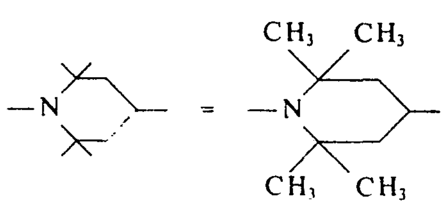

TSS = p-Toluolsulfonsäure
MPS = Methylphosphonsäure $CH_3-P(O)(OH)_2$
MMP = Methyl-methylphosphonat $CH_3-P(O)(OCH_3)(OH)$
DBS = 4-Dodecylbenzolsulfonsäure
MSS = Mesitylensulfonsäure
PSS = techn. $C_{14}-C_{17}$ Paraffinsulfonsäure (Äquivalent-Gewicht 282)
DNND = Dinonylnaphthalindisulfonsäure
NND = techn. Nonylnaphthalindisulfonsäure

| Salz Nr. | Formel | Erweichungs- bzw. Schmelz-temperatur |
|---|---|---|
| 1 | 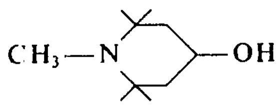 · MMP | 90° |
| 1a | · $^{1}/_{2}$ MPS | 170° |
| 1b | · $^{1}/_{2}$ DNND | 250° |
| 2 | 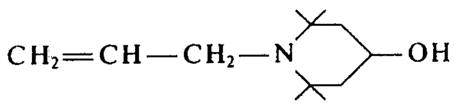 · MMP | Öl |
| 2a | · $^{1}/_{2}$ MPS | 80° |
| 3 | 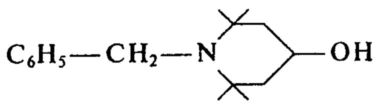 · MPS | 140° |
| 3a | · $^{1}/_{2}$ MPS | 40° |
| 3b | · MMP | 70° |
| 4 | 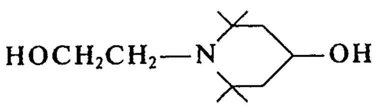 · TSS | 132 bis 134° |
| 4a | · $^{1}/_{2}$ DNND | 98° |
| 5 | 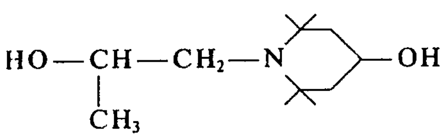 · TSS | 152° |
| 6 | 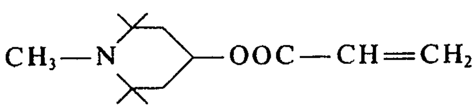 · MMP | Sirup |
| 6a | · $C_4H_9OOC-CH=CH\cdot COOH$ | Öl |
| 6b | · DBS | 94–99° |
| 6c | · TSS | 115° |

Fortsetzung

| Salz Nr. | Formel | Erweichungs- bzw. Schmelz-temperatur |
|---|---|---|
| 6d | 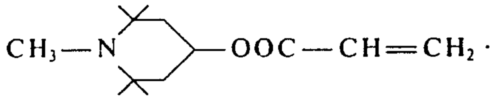 · $CH_3SO_3H$ | 190° |
| 6e | · $^1/_2$ DNND | 69° |
| 7 | 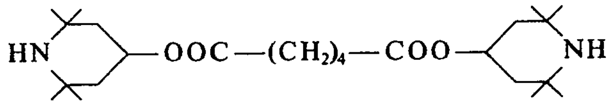 · 2 MMP | 145° |
| 8 | 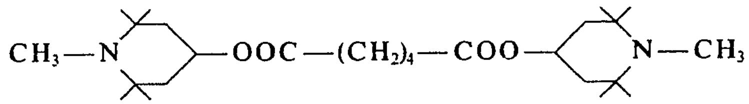 · 2 MMP | Öl |
| 9 | 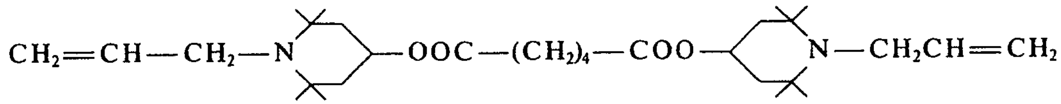 · 2 MMP | 30° |
| 9a | · 2 MPS | 30° |
| 9b | · 2 TSS | 196° |
| 9c | · DNND | 120° |
| 9d | · 2 DBS | 95° |
| 9e | · NND | Öl |
| 10 | 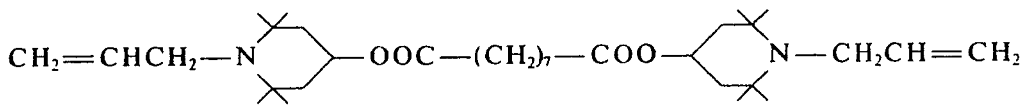 · 2 TSS | Öl |
| 10a | · MPS | Öl |
| 10b | · 2 MMP | flüssig |
| 10c | · 2 MSS | 75° |
| 10d | 2 PSS | Öl |
| 10e | 2 $CH_3SO_3H$ | 62° |

Fortsetzung

| Salz Nr. | Formel | Erweichungs- bzw. Schmelz-temperatur |
|---|---|---|
| 10f | 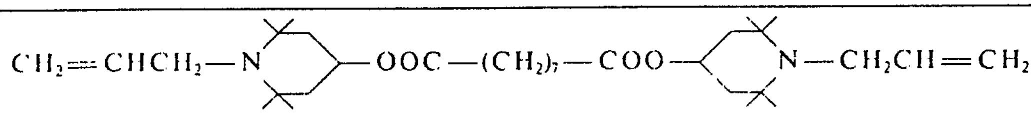 2 $C_2H_5—SO_3H$ | 52° |
| 10g | · 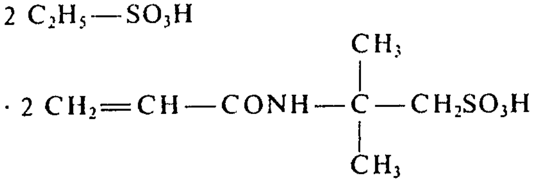 | 56° |
| 10h | · 2 $C_6H_5—SO_3H$ | 48° |
| 10i | · 2 DBS | 55° |
| 10k | · 2 $H_2NSO_3H$ | 40° |
| 10l | · 2 Camphersulfonsäure | 78° |
| 10m | · 2 $C_{14}H_{29}SO_3H$ | Öl |
| 10n | · DNND | 108° |
| 10p | · NND | Öl |
| 11 | 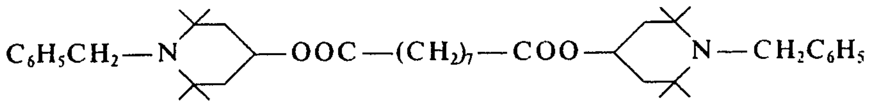 · 2 MMP | Harz |
| 11a | · MPS | Öl |
| 11b | · 2 TSS | 105° |
| 12 | 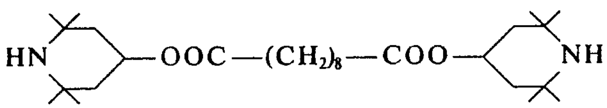 · TSS | Harz |
| 12a | · 2 TTS | 250° |
| 12b | · MPS | 250° (subl.) |
| 12c | · 2 MMP | 250° (subl.) |
| 12d | · 2 $C_4H_9OOC—CH{=}CH—COOH$ | 110° |
| 12e | · 2 $C_6H_{13}OOC—CH{=}CH—COOH$ | 90° |
| 12f | · 2 $C_6H_5—P(O)(OH)_2$ | 80° |
| 12g | · DNND | 160° |

Fortsetzung

| Salz Nr. | Formel | | Erweichungs- bzw. Schmelz-temperatur |
|---|---|---|---|
| 13 | 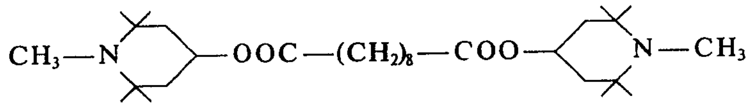 | · DBS | Öl |
| 13a | | · 2 DBS | flüssig |
| 13b | | · TSS | 50° |
| 13c | | · 2 TSS | 75° |
| 13d | | · 2 MPS | Harz |
| 13e | | · 2 $C_{14}H_{29}SO_3H$ | flüssig |
| 13f | | · $C_6H_5P(O)(OH)_2$ | 80° |
| 14 | 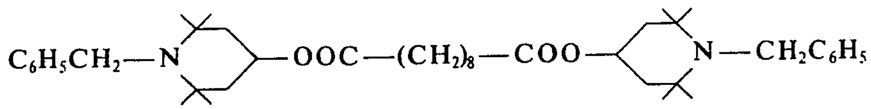 | · 2 MMP | 100° |
| 14a | | · 2 MMP | 98° |
| 14b | | · 2 PSS | Harz |
| 14c | | · 2 DBS | 62° |
| 14d | | · DNND | 120° |
| 14e | | · 2 Vinylsulfonsäure | 160° |
| 14f | | · NND | visk. Öl |
| 14g | | · 1,9 TSS | 90° |
| 14h | | · 1,8 TSS | 90° |
| 14i | | · 1,7 TSS | 88° |

Fortsetzung

| Salz Nr. | Formel | Erweichungs- bzw. Schmelz-temperatur |
|---|---|---|
| 15 | 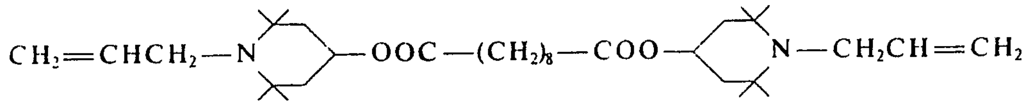 · 2 TSS | 95° |
| 15a | · DNND | 104° |
| 15b | · 2 DBS | 44° |
| 15c | · NND | visk. Öl |
| 15d | · 2 $C_4H_9OOC—CH{=}CH—COOH$ | 50° |
| 15e | · 2 MSS | 78° |
| 16 | 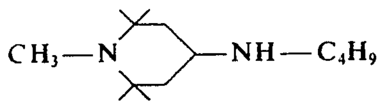 · MPS | 130° |
| 16a | · 2 MMP | 95° |
| 17 | 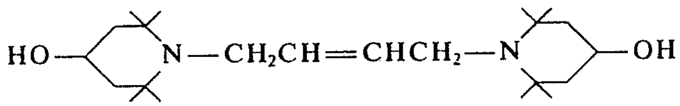 · MPS | 205° |
| 17a | · 2 MMP | 170° |
| 18 | 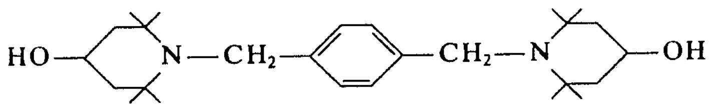 · MPS | 250° |
| 18a | · 2 MMP | 55° |
| 19 | 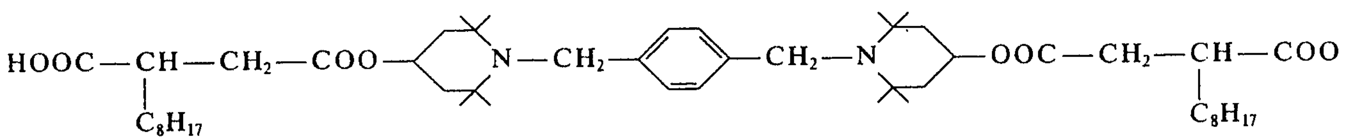 · 2 MMP | 20° |
| 19a | · MPS | 60° |
| 19b | · 2 TSS | 105° |
| 20 | 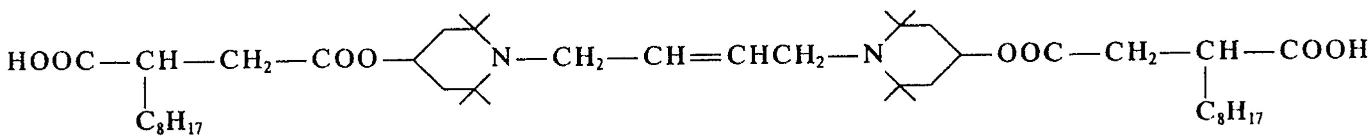 · 2 TSS | 96° |
| 20a | · 2 MMP | 50° |

Fortsetzung

| Salz Nr. | Formel | Erweichungs- bzw. Schmelz- temperatur |
|---|---|---|
| 21 | 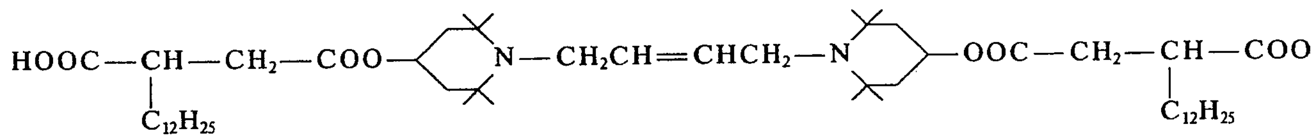 · MPS | 65° |
| 21a | · 2 MMP | Harz |
| 22 | 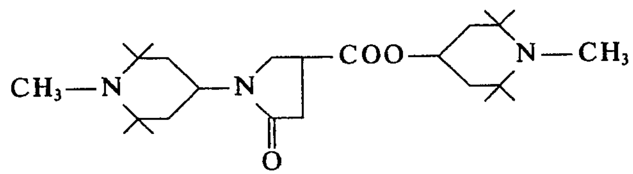 · 2 TSS | 138° |
| 23 | 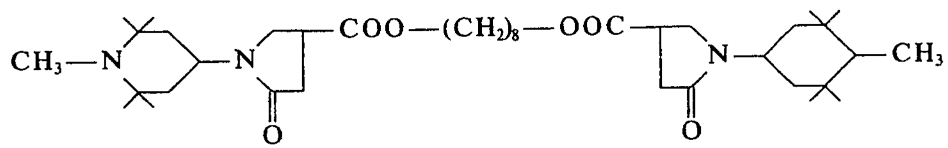 · 2 TSS | §&° |
| 24 | 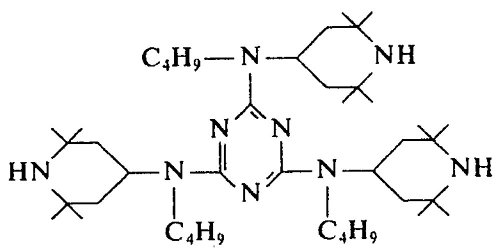 · TSS | 150° |
| 24a | · DBS | 92° |
| 24b | · 2 DBS | 96° |
| 24c | · 3 DBS | 102° |
| 24d | · MPS | 250° |
| 24e | · 1 1/2 MPS | 215° |
| 24f | · 1 1/2 DNND | >250° |

Fortsetzung

| Salz Nr. | Formel | Erweichungs- bzw. Schmelz-temperatur |
|---|---|---|
| 25 | 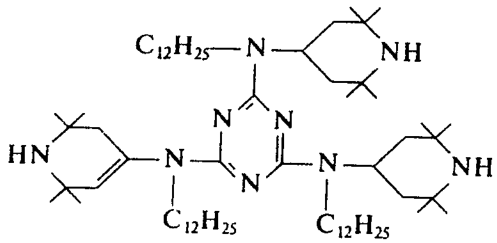 · TSS | 50° |
| 25a | · 2 TSS | 260° |
| 25b | · 3 TSS | 300° |
| 26 | 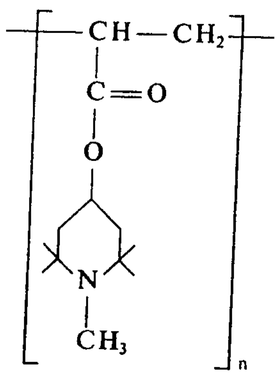 · n TSS | 140° |
| 26a | · n DBS | 96° |
| 27 | 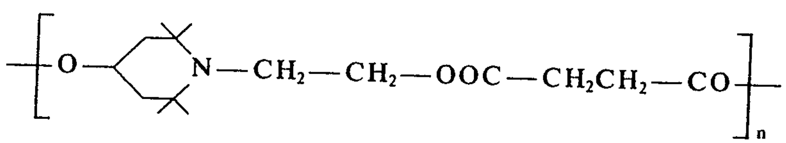 · n TSS | 135° |
| 28 | 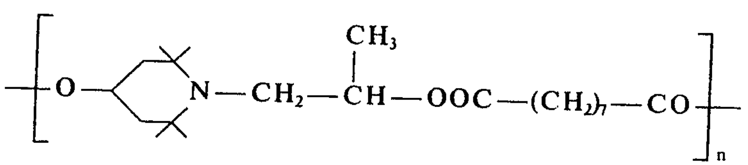 · n TSS | 115° |

Fortsetzung

| Salz Nr. | Formel | Erweichungs- bzw. Schmelztemperatur |
| --- | --- | --- |
| 29 | 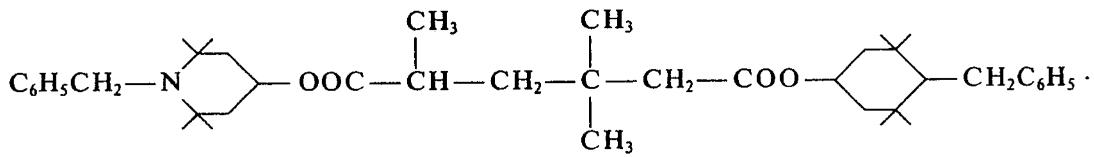 $\cdot$ 2 TSS | 90° |
| 30 | 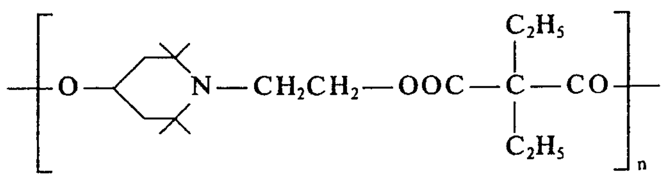 $\cdot \frac{n}{2}$ DNND | 250° |
| 30a | $\cdot$ n TSS | 145° |
| 31 | 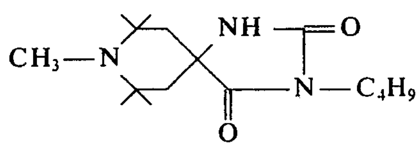 $\cdot$ TSS | 90° |
| 32 | 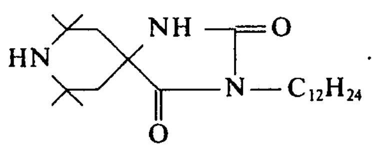 $\cdot$ $CH_3SO_3H$ | 210–218° |
| 33 | 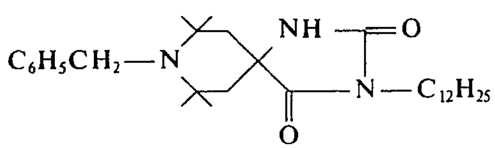 $\cdot$ TSS | 102° |

Fortsetzung

| Salz Nr. | Formel | Erweichungs- bzw. Schmelz-temperatur |
|---|---|---|
| 34 | 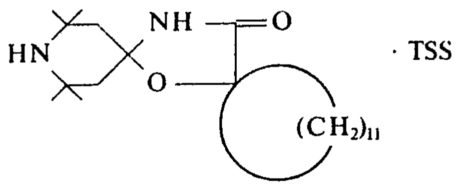 | 230° |
| 35 | 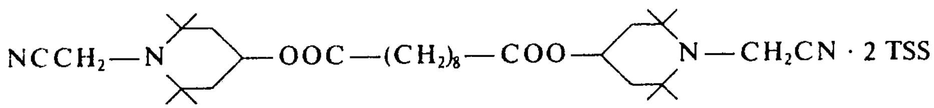 | 105° |
| 36 | 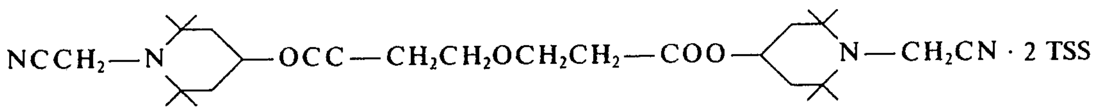 | 114° |
| 37 | 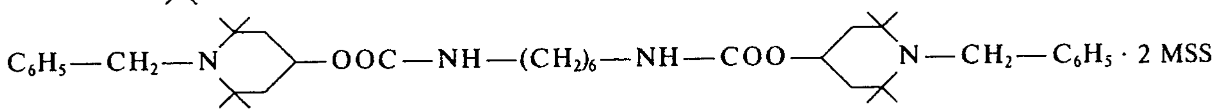 | 132° |
| 38 | 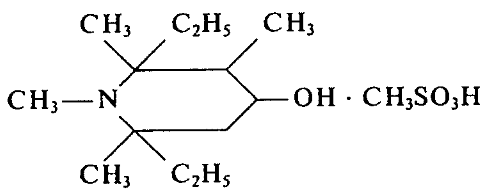 | 60° |
| 39 | 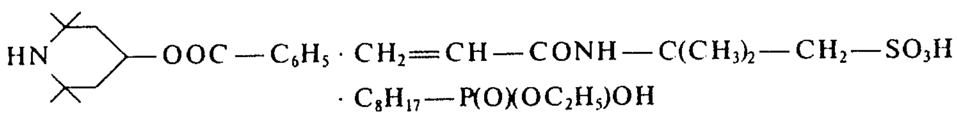 | 71° |
| 39a | $\cdot C_8H_{17}—P(O)(OC_2H_5)OH$ | 65° |
| 39b | $\cdot C_{18}H_{37}—P(O)(OC_2H_5)OH$ | 54° |
| 40 | 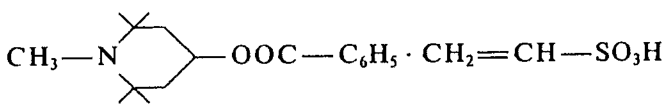 | 71° |

Herstellung von inneren Salzen von Piperidinsulfonsäuren

13,58 g (0,06 Mol) 1,2,2,6,6-Pentamethyl-4-n-butylaminopiperidin und 7,3 g (0,06 Mol) Propansulton werden in 80 ml wasserfreiem Xylol 21 Stunden unter Rühren und in einer $N_2$-Atmosphäre auf 100°C erhitzt.

Beim Abkühlen auf Raumtemperatur entsteht ein Niederschlag, der abgetrennt, in wenig Dichlormethan gelöst und durch Eingießen in 500 ml 2-Butanon unter Rühren bei ca. 0°C erneut gefällt wird. Der Niederschlag wird abfiltriert, mit wenig Diäthyläther nachgewaschen und im Vakuum getrocknet wodurch die als inneres Salz (Betain) vorliegende Sulfonsäure der Struktur

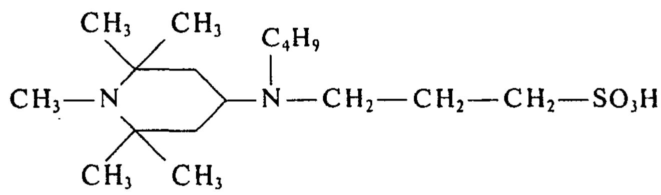

vom Smp. 180—185° erhalten wird (Salz Nr. 50).

In analoger Weise wird aus 14,1 g (0,05 Mol) 1,2,2,6,6-Pentamethyl-4-n-octylaminotetramethylpiperidin und 6,11 g (0,05 Mol) Propansulton in 80 ml Dioxan die als inneres Salz vorliegende Sulfonsäure der Struktur

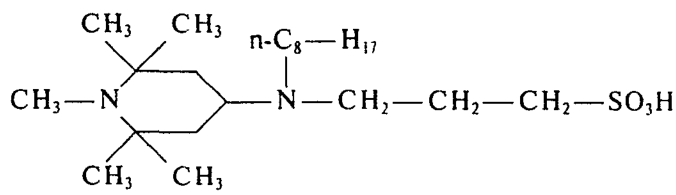

vom Smp. 105—110° erhalten (Salz Nr. 51).

In analoger Weise werden 13 g (0,07 Mol) 1-(2-Hydroxyäthyl)-2,2,6,6-tetramethylpiperidin mit 8,55 g (0,07 Mol) Propansulton bei 110—115° umgesetzt.

Nach dem Abkühlen auf ca. 0—5°C wird der weiße Niederschlag abfiltriert und mit Diäthyläther nachgewaschen.

Anschließend löst man den Niederschlag in 30 ml Chloroform und gießt diese Lösung unter kräftigem Turbinieren in 400 ml eines Diäthyläther-Aceton-Gemisches (3 : 1 Vol). Der weiße Niederschlag wird abgesaugt und im Hochvakuum getrocknet. Die erhaltene Sulfonsäure der Struktur

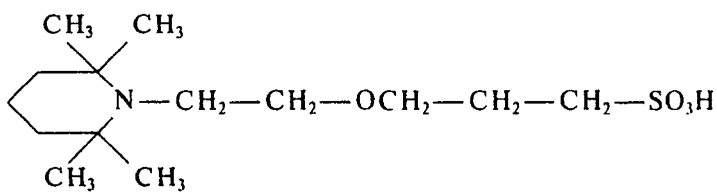

schmilzt bei 235—240° (Salz Nr. 52).

Die für die Härtung der Lacke benötigte Menge an Polyalkylpiperidinsalz richtet sich nach der Art des Lackharzes sowie nach der Härtungstemperatur. Im allgemeinen braucht man etwa 0,1 bis 5 Gew.-% Piperidinsalz, bezogen auf das lösungsmittelfreie Lackharz, vorzugsweise verwendet man 1—2 Gew.-%. In diesen Mengen ist auch eine ausreichende Lichtschutzwirkung der Piperidinsalze gewährleistet. Die Zugabe des Salzes zum Lack geschieht durch einfaches Mischen mit den übrigen Komponenten des Lackes. Man kann das Salz auch in gelöster Form dem Lack zugeben. Dies ist besonders dann von Vorteil, wenn bei der Herstellung des Salzes dieses als Lösung anfällt, so daß seine Isolierung nicht notwendig ist.

Die Lacke enthalten außer dem Lackharz meist ein organisches Lösungsmittel oder Lösungsmittelgemisch, sie können aber auch eine wäßrige Lösung oder Dispersion darstellen oder Lösungsmittelfrei sein. Von besonderem Interesse sind Lacke mit geringem Lösungsmittelanteil, sogenannte ›high solids-Lacke‹. Die Lacke können Klarlacke sein, wie sie z. B. in der Automobilindustrie als Decklacke von Mehrschichten-Anstrichen verwendet werden. Sie können auch Pigmente enthalten, seien es anorganische oder organische Pigmente sowie Metallpigmente für Metalleffekt-Lacke.

Die Lacke können weiterhin kleinere Mengen an speziellen Zusätzen enthalten, wie sie in der Lacktechnologie üblich sind, beispielsweise Verlaufshilfsmittel, Thixotropiemittel oder Antioxydantien. Obwohl die erfindungsgemäß verwendbaren Piperidinsalze hervorragende Lichtschutzmittel sind, kann es in bestimmten Fällen von Vorteil sein, zusätzlich noch andere Lichtschutzmittel mitzuverwenden, wie z. B. solche vom Typ der UV-Absorber oder der organischen Nickelverbindungen, weil dabei

synergistische Effekte auftreten können. Beispiele für verwendbare Klassen von UV-Absorbern sind die Hydroxyphenylbenztriazole, die Hydroxybenzophenone, die Oxalanilide, die Cyanoacrylate und die Hydroxyphenyl-s-triazine. Beispiele für organische Nickelverbindungen sind Nickelsalze von Phosphonsäuremonoestern oder Ni-Komplexe von Bis-phenolen. Besonders bevorzugt ist die Mitverwendung von Hydroxyphenyl-benztriazol-Lichtschutzmitteln, wie z. B. von 2-(2-Hydroxy-3,5-di-t-amylphenyl)-benztriazol oder 2-[2-Hydroxy-3,5-di($\alpha$-dimethylbenzyl)-phenyl]-benztriazol.

Die Lacke werden auf die zu beschichtenden Substrate nach den üblichen Methoden der industriellen Lackierung aufgetragen, beispielsweise durch Streichen, Besprühen oder Tauchen.

Die Lacke werden nach dem Auftragen getrocknet und eingebrannt. Die Härtungstemperatur können bei 80° bis 300°C liegen bei Härtungszeiten von einigen Sekunden bis zu einer Stunde. Für das erfindungsgemäße Verfahren sind vor allem niedrige Härtungstemperaturen von etwa 100° bis 150°C von Interesse, wie sie bei Ausbesserungslackierungen an Automobilen oder bei sonstigen Lackierungen empfindlicher Industrieartikel angewendet werden.

Das erfindungsgemäße Verfahren ist für alle Arten der industriellen Lackierung geeignet, wie z. B. für die Lackierung geeignet, wie z. B. für die Lackierung von Maschinen, Fahrzeugen, Schiffen oder Konstruktionsteilen. Von besonderer Bedeutung ist es für die Fahrzeug-Lackierung. Hierbei kann es sowohl in Einschicht-Lackierung wie Mehrschicht-Lackierung angewendet werden. Von besonderem Interesse ist auch die Anwendung des Verfahrens für die kontinuierliche Beschichtung von Blechen, beispielsweise Stahl- oder Aluminiumblechen, dem sogenannten Coil-Coat-Verfahren.

Im folgenden wird das erfindungsgemäße Verfahren anhand einiger typischer Beispiele näher beschrieben. Hierin bedeuten Teile Gewichtsteile und % Gewichts-%. Die Temperaturen sind in °C angegeben. Die Warenzeichen werden als solche anerkannt.

## Beispiel 1

Ein Klarlack wird durch Mischen der folgenden Bestandteile hergestellt:

53,7 Teile eines hydroxylfunktionellen Acrylharzes (Paraloid OL 42, Rohm & Haas Corp., USA)
19,2 Teile Hexamethoxymethylmelamin (Cymel 301, Amer. Cyanamid, USA)
1,9 Teile Celluloseacetobutyrat (CAB 551 001, Eastman Chem. Corp., USA)
2,9 Teile eines Verlaufhilfsmittels auf Silikonbasis (Byketol Special, Byk-Mallinckrodt GmbH)
0,3 Teile eines Verlaufshilfsmittels auf Basis eines Tensides als 1%ige Lösung in Ethylglykolacetat (Modaflow, Monsanto-Corp.)
10,5 Teile n-Butanol
11,5 Teile Butylacetat.

Dieser Klarlack hat einen Festkörperanteil von 63%. Diesem Klarlack werden die in Tabelle 1 angegebenen Mengen an Härtungskatalysatoren zugesetzt.

Die Lackproben werden auf Glasplatten in einer Schichtdicke (naß) von 50 μm aufgetragen und im Trockenschrank bei der in Tabelle 1 angegebenen Temperatur 30 Minuten gehärtet. Die resultierenden Lackfilme haben eine Schichtdicke von ca. 30 μm. Nach 60 Minuten Lagerung im Normklima (23°C/50% relative Luftfeuchtigkeit) wird die Pendelhärte der Lackfilme nach der Methode von Koenig (DIN 53 157) gemessen. Die Ergebnisse sind in Tabelle I zusammengestellt

Tabelle 1

| Härtungskatalysator und Zusatzmenge*) | | Härtungstemperatur und -Zeit | Pendelhärte nach Koenig (Sekunden) |
|---|---|---|---|
| ohne | | 150°/30 min | klebrig |
| Salz Nr. 1 a | 1% | | 98 |
| | 0,5% | 150°/30 min | 31 |
| Salz Nr. 9 c | 1% | 150°/30 min | 130 |
| Salz Nr. 10 | 1% | 150°/30 min | 156 |
| | 0,5% | 150°/30 min | 148 |
| | 0,5% | 130°/30 min | 114 |
| | 0,5% | 120°/30 min | 67 |
| Salz Nr. 14 | 1% | 150°/30 min | 163 |
| | 0,5% | 150°/30 min | 155 |
| | 0,5% | 130°/30 min | 107 |
| | 0,5% | 120°/30 min | 91 |
| Salz Nr. 3 | 1% | 150°/30 min | 130 |
| Salz Nr. 9 | 1% | 150°/30 min | 97 |
| Salz Nr. 9 c | 1% | 150°/30 min | 130 |
| Salz Nr. 10 d | 1% | 150°/30 min | 140 |
| Salz Nr. 10 m | 1% | 150°/30 min | 140 |
| Salz Nr. 15 a | 1% | 150°/30 min | 135 |
| Salz Nr. 17 | 1% | 150°/30 min | 109 |
| Salz Nr. 19 a | 1% | 150°/30 min | 81 |
| Salz Nr. 27 | 1% | 150°/30 min | 94 |

*) Bezogen auf Festkörpergehalt des Lackes.

Beispiel 2

Der in Beispiel 1 beschriebene Klarlack wird als Decklack einer Zweischicht-Metalleffektlackierung, wie sie in der Automobilindustrie üblich ist, eingesetzt.

Hierzu werden 0,4 mm starke Aluminiumbleche mit einem handelsüblichen Haftgrund (Primer) vorbehandelt und mit einer handelsüblichen Grundierung (Füller) auf Basis eines Epoxid-Polyester-Einbrennlackes beschichtet.

Darauf wird ein silberfarbener Basislack der folgenden Zusammensetzung aufgebracht:

27 Teile eines hitzehärtbaren Polyesterharzes (Polyester L 1850, Dynamit Nobel AG, BRD)
3 Teile eines Melaminharzes (Maprenal RT, Hoechst AG, BRD)
2 Teile eines Celluloseacetobutyrates (CAB 531, Eastman Chem. Corp.)
8 Teile Aluminiumpigment (Alcoa 726, Aluminium Corp, USA)
10 Teile Toluol

7 Teile Xylol
3 Teile Butanol
25 Teile Butylacetat
15 Teile eines aromatischen Lösungsmittelgemisches (Solvesso 100, Esso AG).

Der Basislack wird in einer Schichtdicke von ca. 15 μm (naß) aufgespritzt und auf diesen der Klarlack in einer Schichtdicke von ca. 30–40 μm naß in naß aufgespritzt. Nach kurzer Abluftzeit wird der Lack im Trockenofen 30 min bei 150°C eingebrannt. Die Trockenfilmdicke beträgt ca. 40 μm. Nach 2 Wochen Lagerung im Normklima (23°C/50% relative Luftfeuchtigkeit) werden die Proben in einem Schnellbewitterungsgerät gemäß ASTM G 53–77 bewittert. Als Vergleich wird eine Probe mit p-Toluolsulfonsäure katalysiert ohne zusätzliches Lichtschutzmittel.

Als Kriterium der Stabilisierungswirkung wird der Glanz nach ASTM D 523 (20°-Glanz) herangezogen. Die Resultate sind in Tabelle 2 aufgeführt.

Tabelle 2

| Härtungskatalysator | | 20°-Glanz | |
|---|---|---|---|
| | | unbewittert | 400 h bewittert |
| Salz Nr. 10 | 0,5% | 85 | 59 |
| Salz Nr. 14 | 0,5% | 83 | 56 |
| Toluolsulfonsäure | 0,5% | 86 | 36 |

## Beispiel 3

Der im Beispiel 1 beschriebene Klarlack wird mit jeweils 1 Gew.-% (bezogen auf Festkörper) der in Tabelle 3 aufgeführten erfindungsgemäßen Härtungskatalysatoren versetzt und wie in Beispiel 1 beschrieben auf Glasplatten aufgetragen. Die Härtung erfolgt 30 min bei 150°C. Nach 24 Stunden Lagerung im Normklima (23°C/50% relative Luftfeuchtigkeit) wird die Pendelhärte nach der Methode Koenig (DIN 53 157) gemessen.

Außerdem wird zur Bestimmung der Lagerfähigkeit von jeder katalysierten Lackprobe die Viskosität nach 0, 10, 20, 30, 40 und 50 Tagen Lagerung bei Raumtemperatur mit einem Kegel-Platte-Viskosimeter gemäß DIN 53 229 gemessen. Der in der Tabelle 3 angegebene Wert $T_{40}$ gibt die Zeit in Tagen an, nach welcher die Viskosität des Lackes um 40% gestiegen ist.

Tabelle 3

| Härtungskatalysator (jeweils 1%) | Pendelhärte nach Koenig (Sekunden) | $T_{40}$ (Tage) |
|---|---|---|
| p-Toluolsulfonsäure | 160 | 5 |
| Salz Nr. | | |
| 4 | 164 | 44 |
| 5 | 174 | 47 |
| 6b | 167 | 43 |
| 6c | 170 | 41 |
| 6d | 154 | 40 |
| 10 | 165 | 39 |
| 10c | 172 | 47 |
| 10e | 169 | 36 |
| 10f | 159 | 40 |
| 10g | 170 | 50 |
| 10h | 164 | 38 |
| 10k | 162 | 41 |
| 10l | 167 | 42 |
| 13c | 148 | 39 |
| 15 | 161 | 31 |
| 20 | 168 | 34 |
| 22 | 160 | 42 |
| 23 | 148 | 37 |
| 26 | 148 | 42 |
| 26a | 159 | 47 |
| 29 | 163 | 42 |

Diese Resultate zeigen, daß die erfindungsgemäßen Piperidinsalze in ihrer katalytischen Aktivität der Wirkung von Toluolsulfonsäure nicht nachstehen, jedoch eine wesentlich längere Topfzeit erzielbar ist.

**Patentansprüche**

1. Verfahren zur Härtung von Einbrennlacken in Gegenwart eines Härtungskatalysators, dadurch gekennzeichnet, daß man als Härtungskatalysator entweder

a) ein Säureadditionssalz eines basischen Polyalkylpiperidinderivates, das eine Gruppe der Formel I enthält,

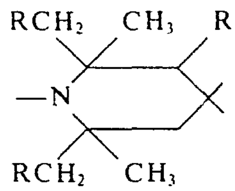

(I)

worin R Wasserstoff oder Methyl ist, und einer anorganischen oder organischen Säure, oder

b) das innere Salz einer Sulfonsäure, die eine Gruppe der Formel I enthält, verwendet.

2. Verfahren gemäß Anspruch 1, dadurch gekennzeichnet, daß man als Härtungskatalysator ein Säureadditionssalz einer Mono- oder Polysulfonsäure, eines sauren Phosphorsäureesters, einer Phosphonsäure oder deren Monoester, der Maleinsäure oder deren Monoester oder der Phthalsäure oder deren Monoester verwendet.

3. Verfahren gemäß Anspruch 2, dadurch gekennzeichnet, daß man als Härtungskatalysator ein Salz eines basischen Polyalkylpiperidinderivates, das eine Gruppe der Formel I enthält, worin R Wasserstoff ist, mit einer Mono- oder Disulfonsäure verwendet.

4. Verfahren gemäß Anspruch 1, dadurch gekennzeichnet, daß man als Härtungskatalysator ein Säureadditionssalz eines basischen Polyalkylpiperidinderivates verwendet, das eine Gruppe der Formel Ia enthält,

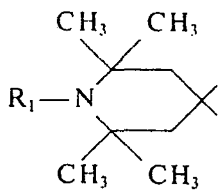

(Ia)

worin $R_1$ $C_1$—$C_4$ Alkyl, Cyanomethyl, $C_3$—$C_5$ Alkenyl oder $C_7$—$C_9$ Aralkyl ist.

5. Verfahren gemäß Anspruch 4, worin $R_1$ Allyl oder Benzyl ist.

6. Verfahren gemäß Anspruch 2, dadurch gekennzeichnet, daß man ein Salz verwendet, das gleichviel Basen- wie Säureäquivalente enthält oder einen geringen Überschuß an Basenäquivalenten enthält.

7. Verfahren gemäß Anspruch 2, dadurch gekennzeichnet, daß man als Härtungskatalysator das innere Salz einer Sulfonsäure von einer der folgenden Formeln verwendet,

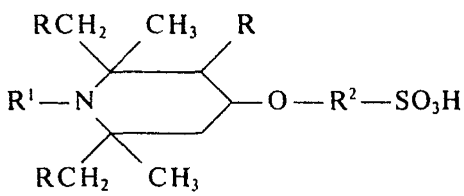

(II)

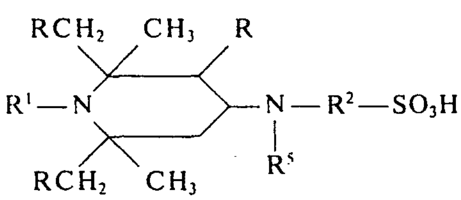

(III)

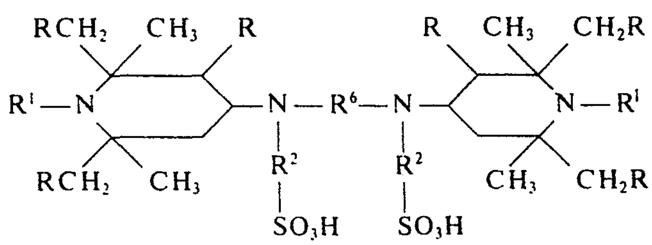

(IV)

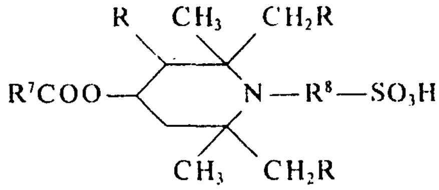

(V)

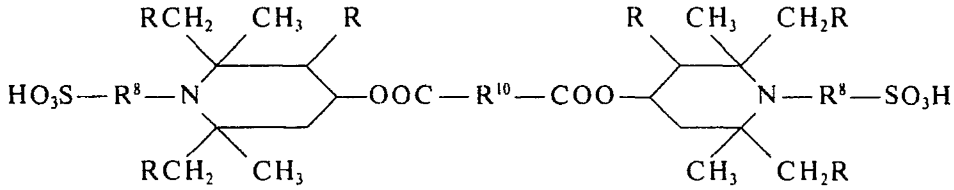

(VI)

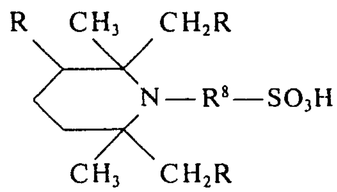

(VII)

worin R Wasserstoff oder Methyl ist,

$R^1$ Wasserstoff, $C_1$–$C_{12}$ Alkyl, $C_3$–$C_8$ Alkenyl, $C_3$–$C_8$ Alkinyl, $C_7$–$C_{12}$ Aralkyl oder $-CH_2CN$ ist,
$R^2$ eine Gruppe $-(CH_2)_3-$ oder $-CH_2-CH(R^3)-CONH-R^4$ darstellt, worin
$R^3$ Wasserstoff oder Methyl und $R^4$ $C_2$–$C_8$ Alkylen bedeutet,
$R^5$ $C_1$–$C_{12}$ Alkyl, $C_5$–$C_7$ Cycloalkyl oder $C_7$–$C_9$ Aralkyl,
$R^6$ $C_2$–$C_{12}$ Alkylen, durch –O– unterbrochenes $C_4$–$C_{10}$ Alkylen, $C_6$–$C_{15}$ Cycloalkylen oder Xylylen,
$R^7$ $C_1$–$C_{18}$ Alkyl, $C_6$–$C_{12}$ Aryl oder Alkaryl, $C_7$–$C_{12}$ Aralkyl oder eine Gruppe $R^{11}-NH-$,
$R^8$ $-(CH_2)_3-$ oder $-CH_2-CH(R^9)-O-(CH_2)_3-$,
$R^9$ Wasserstoff, Methyl, Phenyl, Phenoxymethyl oder Tolyloxymethyl,
$R^{10}$ $C_2$–$C_{12}$ Alkylen, durch –O– unterbrochenes $C_2$–$C_{12}$ Alkylen, $C_6$–$C_{12}$ Arylen, $C_6$–$C_{12}$ Cycloalkylen oder eine Gruppe $-NH-R^{12}-NH-$,
$R^{11}$ $C_1$–$C_{18}$ Alkyl, Cyclohexyl, $C_6$–$C_{12}$ Aryl oder $C_7$–$C_9$ Aralkyl und
$R^{12}$ $C_2$–$C_{12}$ Alkylen, $C_6$–$C_{14}$ Arylen oder $C_6$–$C_{12}$ Cycloalkylen

bedeuten.

8. Verfahren gemäß Anspruch 7, dadurch gekennzeichnet, daß man das innere Salz einer Sulfonsäure der Formel II oder III verwendet, worin $R^1$ Methyl, Allyl oder Benzyl ist und $R^2$ eine Gruppe $-(CH_2)_3-$ darstellt.

9. Verfahren gemäß Anspruch 1, dadurch gekennzeichnet, daß man den Härtungskatalysator in einer Menge von 0,1 bis 5 Gew.-%, vorzugsweise 1 bis 2 Gew.-%, bezogen auf das lösungsmittelfreie Lackharz, verwendet.

10. Verfahren gemäß Anspruch 1, dadurch gekennzeichnet, daß man die Härtung des Lackes bei 80 bis 300°C, vorzugsweise bei 100 bis 150°C, durchführt.

11. Verbindungen der Formel II bis VII gemäß Anspruch 7 und deren innere Salze.

## Claims

1. Process for curing stoving lacquers in the presence of a curing catalyst, characterised in that the curing catalyst used is either

a) an acid addition salt of a basic polyalkylpiperidine derivative which contains a group of the formula I

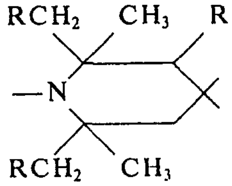

(I)

in which R is hydrogen or methyl, and an inorganic or organic acid, or

b) the inner salt of a sulfonic acid which contains a group of the formula I.

2. Process according to claim 1, characterised in that the curing catalyst used is an acid addition salt of a mono- or polysulfonic acid, of an acid phosphoric acid ester, of a phosphonic acid or a monoester thereof, of maleic acid or a monoester thereof or of phthalic acid or a monoester thereof.

3. Process according to claim 2, characterised in that the curing catalyst used is a salt of a basic polyalkylpiperidine derivative which contains a group of the formula I, in which R is hydrogen, with a mono- or disulfonic acid.

4. Process according to claim 1, characterised in that the curing catalyst used is an acid addition salt of a basic polyalkylpiperidine derivative, which contains a group of the formula Ia

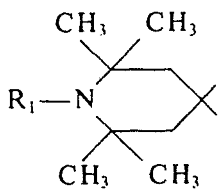

(Ia)

in which $R_1$ is $C_1$—$C_4$-alkyl, cyanomethyl, $C_3$—$C_5$-alkenyl or $C_7$—$C_9$-aralkyl.

5. Process according to claim 4, wherein $R_1$ is allyl or benzyl.

6. Process according to claim 2, characterised in that there is used a salt which contains the same number of base equivalents as acid equivalents, or contains a slight excess of base equivalents.

7. Process according to claim 2, characterised in that the curing catalyst used is the inner salt of a sulfonic acid of one of the following formulae

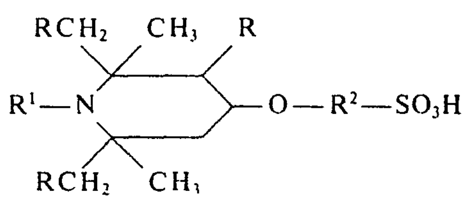

(II)

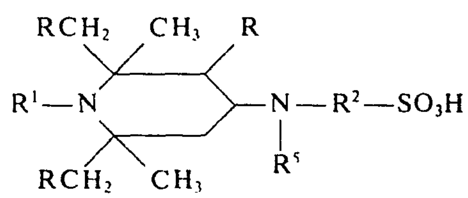

(III)

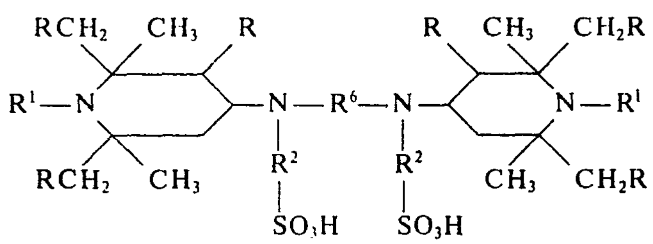

(IV)

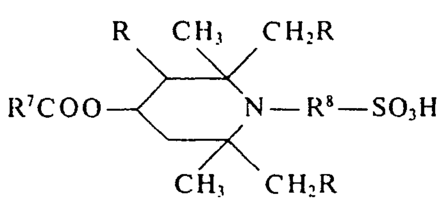

(V)

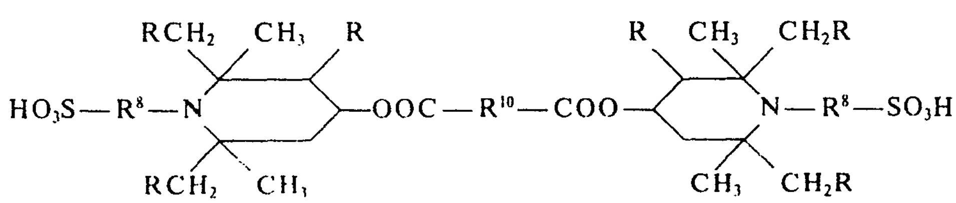

(VI)

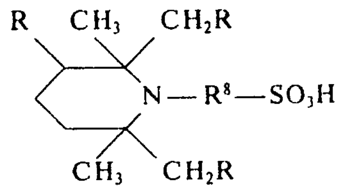

(VII)

wherein

R is hydrogen or methyl,
$R^1$ is hydrogen, $C_1$–$C_{12}$-alkyl, $C_3$–$C_8$-alkenyl, $C_3$–$C_8$-alkynyl, $C_7$–$C_{12}$-aralkyl or $-CH_2CN$,
$R^2$ is a $-(CH_2)_3-$ or $-CH_2-CH(R^3)-CONH-R^4$-group, in which $R^3$ is hydrogen or methyl and $R^4$ is $C_2$–$C_8$-alkylene,
$R^5$ is $C_1$–$C_{12}$-alkyl, $C_5$–$C_7$-cycloalkyl or $C_7$–$C_9$-aralkyl,
$R^6$ is $C_2$–$C_{12}$-alkylene, $C_4$–$C_{10}$-alkylene which is interrupted by $-O-$, $C_6$–$C_{15}$-cycloalkylene or xylylene,
$R^7$ $C_1$–$C_{18}$-alkyl, $C_6$–$C_{12}$-aryl or alkaryl, $C_7$–$C_{12}$-aralkyl or an $R^{11}-NH$-group,
$R^8$ is $-(CH_2)_3-$ or $-CH_2-CH(R^9)-O-(CH_2)_3-$,
$R^9$ is hydrogen, methyl, phenyl, phenoxymethyl or tolyloxymethyl,
$R^{10}$ is $C_2$–$C_{12}$-alkylene, $C_2$–$C_{12}$-alkylene interrupted by $-O-$, $C_6$–$C_{12}$-arylene, $C_6$–$C_{12}$ cycloalkylene or an $-NH-R^{12}-NH$-group,
$R^{11}$ is $C_1$–$C_{18}$-alkyl, cyclohexyl, $C_6$–$C_{12}$-aryl or $C_7$–$C_9$-aralkyl, and
$R^{12}$ is $C_2$–$C_{12}$-alkylene, $C_6$–$C_{14}$-arylene or $C_6$–$C_{12}$-cycloalkylene.

8. Process according to claim 7, characterised in that there is used the inner salt of a sulfonic acid of the formula II or III in which $R^1$ is methyl, allyl or benzyl, and $R^2$ is a $-(CH_2)_3$-group.

9. Process according to claim 1, characterised in that the curing catalyst is used in an amount of 0.1 to 5% by weight, preferably 1 to 2% by weight, relative to the solvent-free lacquer resin.

10. Process according to claim 1, characterised in that the lacquer is cured at 80 to 300°C, preferably at 100 to 150°C.

11. Compounds of the formulae II to VII according to claim 7 and the inner salts thereof.

## Revendications

1. Procédé pour durcir des peintures et vernis à cuire, en présence d'un catalyseur de durcissement, procédé caractérisé en ce qu'on utilise, comme catalyseur de durcissement, soit:

a) un sel d'addition d'acide qui dérive d'un composé polyalkyl-pipéridinique basique contenant un radical répondant à la formule (I):

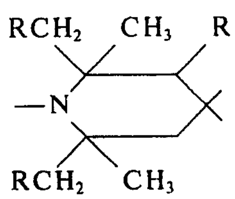

(I)

dans laquelle R représente l'hydrogène ou un radical méthyle, et d'un acide minéral ou organique, soit

b) le sel interne d'un acide sulfonique contenant un radical de formule (I).

2. Procédé selon la revendication 1 caractérisé en ce qu'on utilise, comme catalyseur de durcissement, un sel d'addition dérivant d'un acide mono- ou poly-sulfonique, d'un ester phosphorique acide, d'un acide phosphonique, d'un monoester d'acide phosphonique, de l'acide maléique, d'un monoester de l'acide maléique, de l'acide phtalique ou d'un monoester de l'acide phtalique.

3. Procédé selon la revendication 2 caractérise en ce qu'on utilise, comme catalyseur de durcissement, un sel dérivant d'un composé polyalkyl-pipéridinique basique contenant un radical de formule (I) dans lequel R désigne l'hydrogène, et d'un acide monosulfonique ou disulfonique.

4. Procédé selon la revendication 1 caractérisé en ce qu'on utilise, comme catalyseur de durcissement, un sel d'addition qui dérive d'un composé polyalkyl-pipéridinique basique contenant un radical répondant à la formule (Ia):

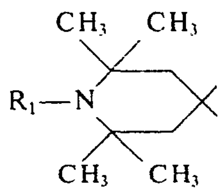

(Ia)

dans laquelle $R_1$ représente un alkyle en $C_1-C_4$, un cyanométhyle, un alcényle en $C_3-C_5$ ou un aralkyle en $C_7-C_9$.

5. Procédé selon la revendication 4 caractérisé en ce que $R_1$ représente un radical allyle ou benzyle.

6. Procédé selon la revendication 2 caractérisé en ce qu'on utilise un sel qui contient, en équivalents, autant de base que d'acide ou qui contient un léger excès, en équivalents, de la base.

7. Procédé selon la revendication 2 caractérisé en ce qu'on utilise, comme catalyseur de durcissement, le sel interne d'un acide sulfonique répondant à l'une des formules suivantes:

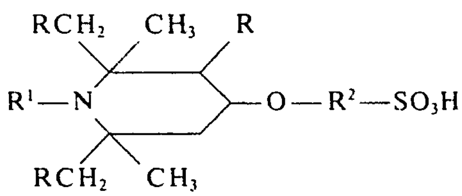

(II)

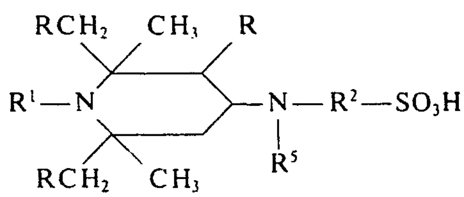

(III)

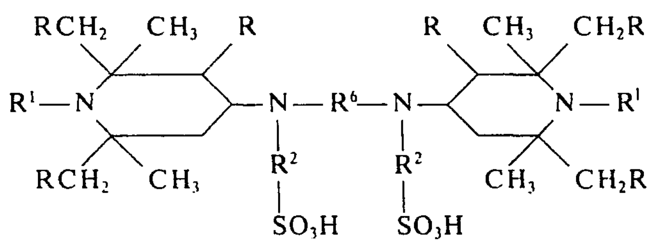

(IV)

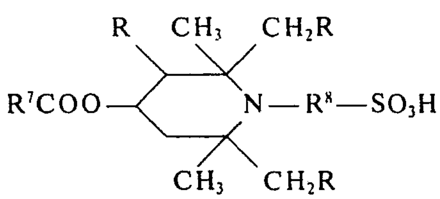

(V)

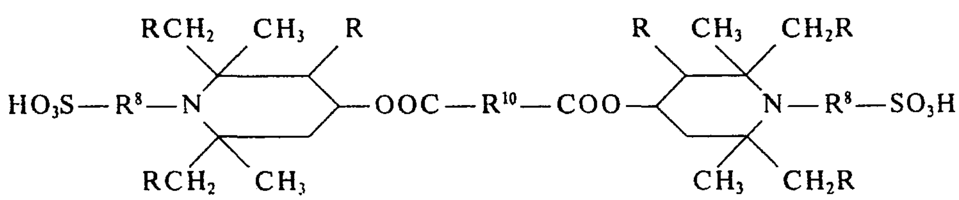

(VI)

et

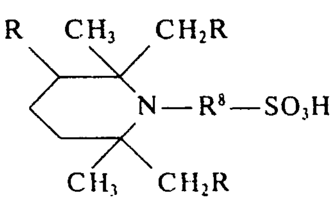

(VII)

dans lesquelles:

$R^1$ représente l'hydrogène, un alkyle en $C_1-C_{12}$, un alcényle en $C_3-C_8$, un alcynyle en $C_3-C_8$, un aralkyle en $C_7-C_{12}$ ou un radical $-CH_2CN$,
$R^2$ représente un radical $-(CH_2)_3-$ ou un radical $-CH_2-CH(R^3)-CONH-R^4-$ dans lequel $R^3$ représente l'hydrogène ou un méthyle et $R^4$ un alkylène en $C_2-C_8$,
$R^5$ représente un alkyle en $C_1-C_{12}$, un cycloalkyle en $C_5-C_7$ ou un aralkyle en $C_7-C_9$,
$R^6$ représente un alkylène en $C_2-C_{12}$, un alkylène en $C_4-C_{10}$ interrompu par $-O-$, un cycloalkylène en $C_6-C_{15}$ ou un xylylène,
$R^7$ représente un alkyle en $C_1-C_{18}$, un alkylaryle ou un aryle en $C_6-C_{12}$, un aralkyle en $C_7-C_{12}$ ou un radical $R^{11}-NH-$,
$R^8$ représente un radical $-(CH_2)_3-$ ou un radical $-CH_2-CH(R^9)-O-(CH_2)_3-$,
$R^9$ représente l'hydrogène, un méthyle, un phényle, un phénoxyméthyle ou un tolyloxyméthyle,
$R^{10}$ représente un alkylène en $C_2-C_{12}$, un alkylène en $C_2-C_{12}$ interrompu par $-O-$, un arylène en $C_6-C_{12}$, un cycloalkylène en $C_6-C_{12}$ ou un radical $-NH-R^{12}-NH-$,
$R^{11}$ représente un alkyle en $C_1-C_{18}$, un cyclohexyle, un aryle en $C_6-C_{12}$ ou un aralkyle en $C_7-C_9$, et
$R^{12}$ représente un alkylène en $C_2-C_{12}$, un arylène en $C_6-C_{14}$ ou un cyclo-alkylène en $C_6-C_{12}$.

8. Procédé selon la revendication 7 caractérisé en ce qu'on utilise le sel interne d'un acide sulfonique de formule (II) ou (III) dans lequel $R^1$ représente un radical méthyle, allyle ou benzyle et $R^2$ un radical $-(CH_2)_3-$.

9. Procédé selon la revendication 1 caractérisé en ce qu'on utilise le catalyseur de durcissement en une quantité de 0,1 à 5% en poids, de préférence de 1 à 2% en poids, par rapport à la résine pour peinture sans solvant.

10. Procédé selon la revendication 1 caractérisé en ce qu'on effectue le durcissement de la peinture à une température de 80 à 300°C, de préférence de 100 à 150°C.

11. Composés de formules (II) à (VII) qui ont été définis à la revendication 7 et sels internes de ces composés.